# EUROPEAN PATENT SPECIFICATION

(11) **EP 2 648 734 B1**
(45) Date of publication and mention of the grant of the patent: **13.02.2019**
(21) Application number: 11847338.8
(22) Date of filing: 06.12.2011
(51) Int. Cl.: A61K 31/475, A61K 31/282, A61K 31/513, A61K 31/704, A61K 31/7048, A61K 38/28, A61K 39/395, A61K 45/00, A61P 35/00, A61P 35/04, C07K 16/28, C07K 16/32

(54) **METHODS OF METABOLIC TARGETING CANCER CELLS USING CHEMO- AND IMMUNOTHERAPY FOR TREATING CANCER**
VERFAHREN ZUM METABOLISCHEN TARGETING VON KREBSZELLEN MIT CHEMO- UND IMMUNTHERAPIEN ZUR BEHANDLUNG VON KREBS
PROCÉDÉS DE CIBLAGE MÉTABOLIQUE DE CELLULES CANCÉREUSES UTILISANT LA CHIMIOTHÉRAPIE ET L'IMMUNOTHÉRAPIE POUR LE TRAITEMENT DU CANCER

(30) Priority: 06.12.2010 US 420208 P
(43) Date of publication of application: 16.10.2013
(73) Proprietor: Cure Cancer Worldwide LLC, Tucson, AZ 85749 (US)
(72) Inventor: TSANG, Tom, C, Zhenjiang Jiangsu (CN); MEADE-TOLLIN, Linda, Tucson AZ 85704 (US); PENNINGTON, Michael, E, Tucson AZ 85719 (US)
(74) Representative: Viering, Jentschura & Partner mbB Patent- und Rechtsanwälte
(86) International application number: PCT/CN2011/002035
(87) International publication number: WO 2012/075679

(56) References cited:
- B. MARTIN-CASTILLO ET AL: "Incorporating the antidiabetic drug metformin in HER2-positive breast cancer treated with neo-adjuvant chemotherapy and trastuzumab: an ongoing clinical-translational research experience at the Catalan Institute of Oncology", ANNALS OF ONCOLOGY, vol. 21, no. 1, 1 January 2010 (2010-01-01), pages 187-189, XP055103916, ISSN: 0923-7534, DOI: 10.1093/annonc/mdp494
- APOSTOLIA M. TSIMBERIDOU ET AL: "Mylotarg, fludarabine, cytarabine (ara-C), and cyclosporine (MFAC) regimen as post-remission therapy in acute myelogenous leukemia", CANCER CHEMOTHERAPY AND PHARMACOLOGY, vol. 52, no. 6, 1 December 2003 (2003-12-01), pages 449-452, XP055103236, ISSN: 0344-5704, DOI: 10.1007/s00280-003-0671-3
- H. A. HIRSCH ET AL: "Metformin Selectively Targets Cancer Stem Cells, and Acts Together with Chemotherapy to Block Tumor Growth and Prolong Remission", CANCER RESEARCH, vol. 69, no. 19, 14 September 2009 (2009-09-14), pages 7507-7511, XP055101751, ISSN: 0008-5472, DOI: 10.1158/0008-5472.CAN-09-2994
- EDUARDO LASALVIA-PRISCO ET AL: "Insulin-induced enhancement of antitumoral response to methotrexate in breast cancer patients", CANCER CHEMOTHERAPY AND PHARMACOLOGY, vol. 53, no. 3, 1 March 2004 (2004-03-01), pages 220-224, XP055102945, ISSN: 0344-5704, DOI: 10.1007/s00280-003-0716-7
- S AYRE: "Insulin, chemotherapy, and the mechanisms of malignancy: the design and the demise of cancer", MEDICAL HYPOTHESES, vol. 55, no. 4, 1 October 2000 (2000-10-01), pages 330-334, XP055101797, ISSN: 0306-9877, DOI: 10.1054/mehy.2000.1063
- ALEJANDRO VAZQUEZ-MARTIN ET AL: "The anti-diabetic drug metformin suppresses self-renewal and proliferation of trastuzumab-resistant tumor-initiating breast cancer stem cells", BREAST CANCER RESEARCH AND TREATMENT, KLUWER ACADEMIC PUBLISHERS, BO, vol. 126, no. 2, 11 May 2010 (2010-05-11), pages 355-364, XP019890345, ISSN: 1573-7217, DOI: 10.1007/S10549-010-0924-X
- R. ROSELL ET AL: "Randomized phase II study of cetuximab plus cisplatin/vinorelbine compared with cisplatin/vinorelbine alone as first-line therapy in EGFR-expressing advanced non-small-cell lung cancer", ANNALS OF ONCOLOGY, vol. 19, no. 2, 10 January 2008 (2008-01-10), pages 362-369, XP055103213, ISSN: 0923-7534, DOI: 10.1093/annonc/mdm474
- Anonymous: "Insulin Potentiation Therapy", , 11 January 2008 (2008-01-11), XP055103255, Retrieved from the Internet: URL:http://www.cancer.org/treatment/treatm entsandsideeffects/complementaryandalterna tivemedicine/pharmacologicalandbiologicalt reatment/insulin-potentiation-therapy [retrieved on 2014-02-19]
- Robert Baratz: "Why You Should Stay Away from Insulin Potentiation Therapy", , 31 August 2013 (2013-08-31), XP055103256, Retrieved from the Internet: URL:http://www.quackwatch.org/01QuackeryRe latedTopics/Cancer/ipt.html [retrieved on 2014-02-19]
- "Correction: Metformin Selectively Targets Cancer Stem Cells, and Acts Together with Chemotherapy to Block Tumor Growth and Prolong Remission", CANCER RESEARCH, vol. 69, no. 22, 3 November 2009 (2009-11-03), pages 8832-8833, XP055101749, ISSN: 0008-5472, DOI: 10.1158/0008-5472.CAN-09-3869
- REN QING ET AL.: 'Trastuzumab combined with chemotherapy in the treatment for 40 cases with er-2-overexpressing metastatic breast cancer (Chinese)' JOURNAL OF ONCOLOGY vol. 16, no. 3, 2010, pages 175 - 177, XP008167947
- WU ZHIYONG ET AL.: 'Effect of combined treatment of trastuzumab and etoposide with different sequences on human breast cancer cells in vitro (Chinese)' CHINESE JOURNAL OF DRUG APPLICATION AND MONITORING vol. 7, no. 2, April 2010, pages 85 - 87, XP008167914
- FERNANDO M. SAFDIE ET AL.: 'Fasting and cancer treatment in humans: A case series report' AGING vol. 1, no. 12, December 2009, pages 1 - 18, XP055102665
- Anonymous: "Trexall, Rheumatrex (methotrexate) dosing, indications, interactions, adverse effects, and more.", reference.medscape.com, 1 January 2016 (2016-01-01), pages 1-3, XP055244592, Retrieved from the Internet: URL:http://reference.medscape.com/drug/tre xall-methotrexate-343201#0 [retrieved on 2016-01-25]
- Anonymous: "doxorubicin dosing, indications, interactions, adverse effects, and more", reference.medscape.com, 1 January 2016 (2016-01-01), pages 1-1, XP055244595, Retrieved from the Internet: URL:http://reference.medscape.com/drug/dox orubicin-342120#0 [retrieved on 2016-01-25]
- C Damyanov ET AL: "Low dose chemotherapy in combination with insulin for the treatment of advanced metastatic tumors. Preliminary experience", Journal of B.U.ON. : official journal of the Balkan Union of Oncology, 1 October 2009 (2009-10-01), page 711, XP55102947, Greece Retrieved from the Internet: URL:http://www.ncbi.nlm.nih.gov/pubmed/201 48468
- Chien-Chung Lin ET AL: "Abstract 71: Metformin inhibits lung cancer cell growth and angiogenesis in vitro and in vivo: Effect on IL6-Stat3 pathway", CANCER RESEARCH, vol. 70, no. 8 Supplement, 15 April 2010 (2010-04-15), pages 71-71, XP055413848, US ISSN: 0008-5472, DOI: 10.1158/1538-7445.AM10-71
- XUE Z-X ET AL: "Metformin inhibits cell proliferation and migration in gastric cancer cell line AGS", SHIJIE HUAREN XIAOHUA ZAZHI - WORLD CHINESE JOURNAL OFDIGESTOLOGY, SHIJIE WEI-CHANGBINGXUE ZAZHISHE, TAIYUAN, CN, vol. 18, no. 19, 8 July 2010 (2010-07-08), pages 1974-1978, XP009500665, ISSN: 1009-3079
- SPIERINGS L E ET AL: "Metformin Use During Treatment of Potentially Curable Esophageal Cancer Patients is not Associated with Better Outcomes", ANNALS OF SURGICAL ONCOLOGY, RAVEN PRESS, NEW YORK, NY, US, vol. 22, no. 3, 8 September 2015 (2015-09-08), pages 766-771, XP035897424, ISSN: 1068-9265, DOI: 10.1245/S10434-015-4850-3 [retrieved on 2015-09-08]
- Ke Zou ET AL: "Pretreatment with insulin enhances anticancer functions of 5-fluorou-racil in human esophageal and colonic cancer cells", Acta Pharmacologica Sinica, vol. 28, no. 5, 1 May 2007 (2007-05-01), pages 721-730, XP055102950, ISSN: 1671-4083, DOI: 10.1111/j.1745-7254.2007.00554.x
- ZWEIG ROCHA G ET AL: "279 AMPK activators act together with paclitaxel to block tumour growth", EUROPEAN JOURNAL OF CANCER. SUPPLEMENT, PERGAMON, OXFORD, GB, vol. 8, no. 5, 1 June 2010 (2010-06-01), page 73, XP027105514, ISSN: 1359-6349 [retrieved on 2010-06-01]

## Description

### BACKGROUND

Cancer is a leading cause of death worldwide. In the United States alone, over 1.3 million people are diagnosed with cancer each year and over 500,000 die. Due to the high incidence and mortality rate, research efforts have focused on improving treatment options for those who are diagnosed with cancer, but a cure has been elusive, especially in later stages of the disease.

Treatment options are determined by the type and stage of the cancer and the patient's overall health. Several modalities of treatment are available, including surgery, chemotherapy, radiation therapy, targeted therapy and immunotherapy. Primary tumors in early stages are sometimes treated by surgery followed by radiation therapy, but in general, most cancers involve treatment with chemotherapy.

Chemotherapy drugs are administered systemically and attack all cells of the body, not just cancer cells. Some chemotherapy drugs are used alone for treating cancer, but often several drugs may be combined, known as combination chemotherapy. Further, chemotherapy is often used together with other modalities of treatment such as surgery, radiation therapy, targeted therapy and immunotherapy.

Because chemotherapy drugs are usually given at the maximum tolerated dose, frequent and dramatic toxicities result that compromise the quality of life and the immune response toward opportunistic infection and toward the cancer itself. These toxicities manifest themselves as side effects such as nausea, hair loss (alopecia), hematopoietic toxicity, decreased mobilization of hematopoietic progenitor cells from bone marrow into the peripheral blood, anemia, myelosuppression, pancytopenia, thrombocytopenia, neutropenia, lymphopenia, leucopenia, stomatitis, esophagitis, heart damage, nervous system damage, lung damage, reproductive system damage, liver damage, kidney and urinary system damage, fatigue, constipation, diarrhea, loss of appetite, headache and muscle pain. These side effects often limit the dose of the chemotherapy agents that can be administered and the frequency at which they can be given.

Acute myelosuppression as a consequence of chemotherapy is well recognized as a dose-limiting factor in cancer treatment. Although other normal tissues may also be adversely affected, bone marrow is particularly sensitive to proliferation-specific treatments such as chemotherapy or radiation therapy. Repeated or high dose cycles of chemotherapy may result in severe stem cell depletion leading to long-term immune suppression or exhaustion. Immune suppression and other side effects often limit the dose or frequency at which treatments may be given, interfere with other treatments that are used in combination with chemotherapy, and otherwise cause interruption of cancer treatments and allow the disease to progress.

Ayre et al. disclose the treatment of cancer by means of a combination of insulin and chemotherapy (Ayre et al., Med Hypotheses. 2000 Oct;55(4):330-4).

However, there is still a need for improved regimens for use in the treatment of cancer that decrease side effects of chemotherapy and increase the efficacy of chemotherapy.

### SUMMARY

A metabolic targeting chemo-immunotherapy regimen for use in the treatment of cancer according to the appended claims is provided herein, the metabolic targeting chemo-immunotherapy regimen comprising cetuximab at a dose of 20 mg ; insulin at a dose of 0.1 to 0.5 units/kg to reduce blood glucose level to 2.2 to 2.8 mmol/L; and a therapeutically effective dose of one or more chemotherapeutic agents, to be administered at a blood glucose level of 2.2 to 2.8 mmol/L. The blood glucose level is reduced by administering a dose of insulin, or a combination thereof with fasting. In one embodiment, the regimen may further comprise administering one or more booster doses of cetuximab. The one or more booster doses may be administered at any interval, including, but not limited to, an interval of two weeks.

The one or more chemotherapeutic agents are selected from the group consisting of alkylating agents, antimetabolites, anti-tumor antibiotics, topoisomerase inhibitors, mitotic inhibitors hormone therapy, glycolysis inhibitors, targeted therapeutics and immunotherapeutics.

The metabolic targeting chemo-immunotherapy regimens described herein are used for treating a cancer selected from the group consisting of bone cancer, bladder cancer, brain cancer, breast cancer, cancer of the urinary tract, carcinoma, cervical cancer, colon cancer, esophageal cancer, gastric cancer, head and neck cancer, hepatocellular cancer, liver cancer, lung cancer, lymphoma and leukemia, melanoma, ovarian cancer, pancreatic cancer, pituitary cancer, prostate cancer, rectal cancer, renal cancer, sarcoma, testicular cancer, thyroid cancer, and uterine cancer.

### BRIEF DESCRIPTION OF THE DRAWINGS

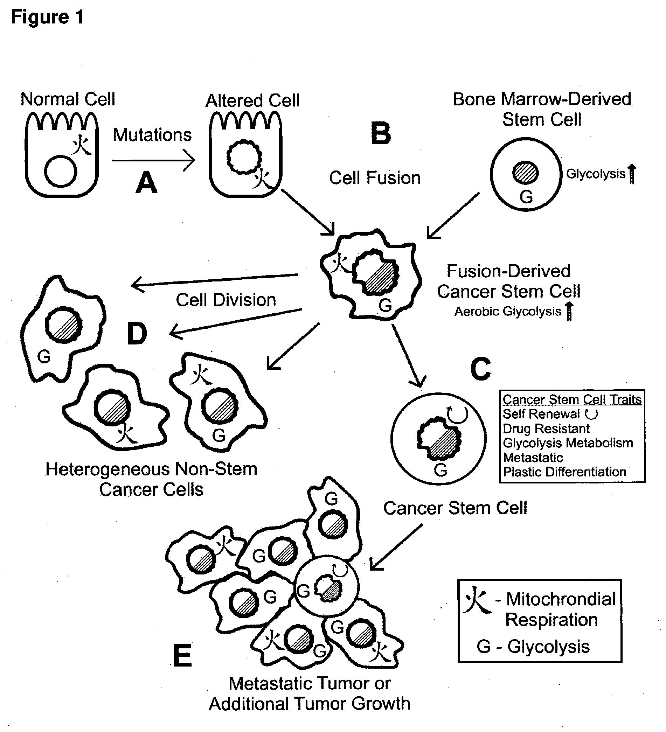

Figure 1 is a schematic showing multi-step carcinogenesis of cancer stem cells (CSC) through cell fusion. A model for cancer stem cells been derived by fusion between genetically altered cells and bone-marrow-derived stem cells is shown by several steps. First, in step (A), genetic mutations lead to an altered hyperplasia cell phenotype leading to a benign neoplasm and local tissue damage. In step (B), bone marrow-derived stem cells, which use glycolysis as its metabolic energy source, are recruited to damage tissue and fuse with an altered cell. The progeny of the fusion will exhibit the hallmark of aneuploidy. In step (C), genetic mutations from the altered cells and epigenetic traits from the bone marrow-derived stem cells are combined to form a cancer stem cell. Stem cell traits include: self renewal, drug resistance, plasticity, glycolysis based metabolism (i.e., the Warburg effect), and capability to move about the body and forming metastasis. In step (D), Stem cell-like plasticity enables the cancer stem cell to divide and differentiate into a heterogeneous cancer cell population with different metabolic phenotypes. In step E, cancer stem cells are embedded within a primary and/or metastatic tumor. They have the ability for self renewal. Cancer stem cell survival from therapies allows regrowth and reoccurrence of cancer.

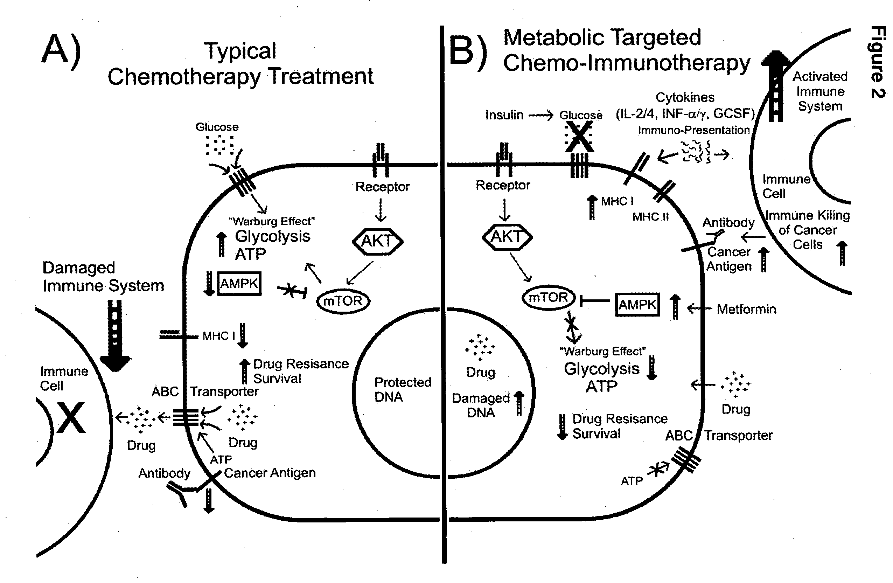

Figure 2 is a schematic diagram illustrating comparisons of typical chemotherapy treatment versus metabolic targeted chemo-immunotherapy on cancer stem cells, according to the embodiments described herein. Cancer stem cells have a glycolysis based metabolism (i.e., the Warburg effect). Panel A shows that current chemotherapy protocols are administered without modifying the metabolism of the cancer stem cells. Chemotherapy given under typical conditions has glucose present for ATP production through glycolysis. ATP is consumed by ABC transporters which protect the cancer stem cells by exporting drugs. At the same time, standard high dose chemotherapy damages the immune system causing diminished effectiveness of antibody dependent cell toxicity and other immunotherapies, some of which utilize the major histocompatability complex I (MHC I) for recognition of tumor cells. Panel B shows that a combined therapy of metabolic targeting, immunotherapy and chemotherapy kills cancer stem cells. Insulin lowers blood sugar levels and depriving cancer stem cells of necessary glucose for ATP generation through glycolysis. In addition, Metformin disrupts signaling through the AKT pathway to further limit ATP production via glycolysis. ABC transporters starved of ATP are unable to pump drugs out of the cell leading to DNA damage. Lower, yet more frequent, doses of chemotherapy spares the immune system allowing antibodies and cytokines to enhance immune killing of cancer cells. Cytokine treatment of cancer stem cells activates the expression of MHC II which in turn presents tumor antigens to immune cells promoting additional immune responses against cancer cells.

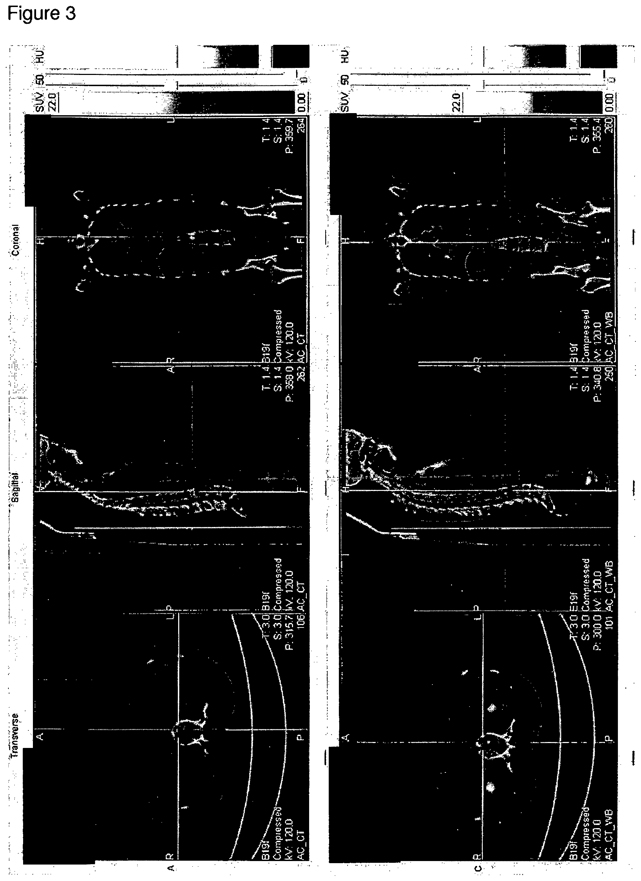

Figure 3 shows representative PET/CT scans for an exemplar patient (Patient 4) receiving the metabolic targeting chemo-immunotherapy described in the embodiments herein. Said patient had extensive disease involvement in the L2 vertebrae with high PET SUV in the before treatment PET/CT scan (bottom panel). A follow up PET/CT scan post treatment showed nearly full regression of L2 vertebrae and bone regeneration (top panel).

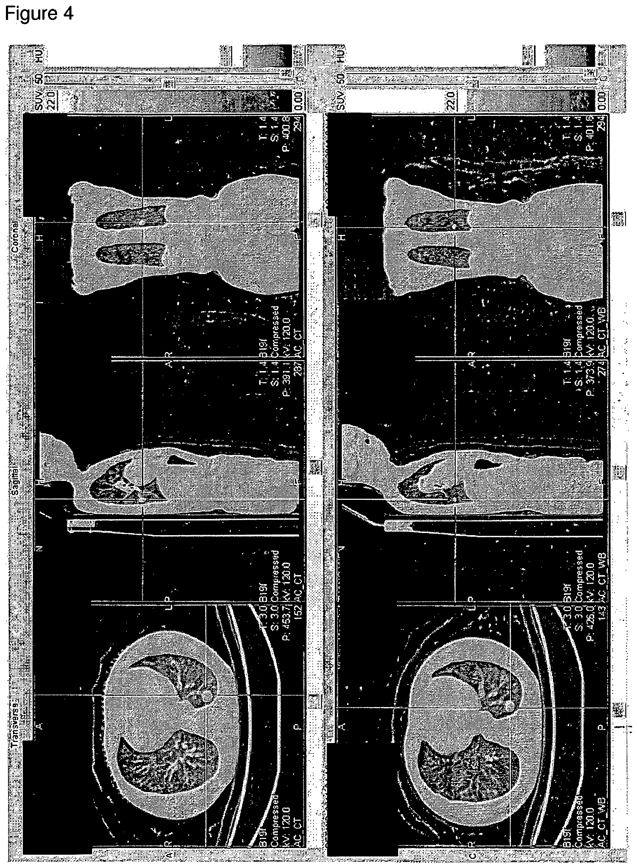

Figure 4 shows a lung lesion from Patient 4. The mass measured 1.3 X 1.5 X 0.8 cm in the pre-treatment PET/CT scan (Figure 4, bottom panel). SUV value was low, 1.5, indicating low glucose uptake in the lesion. A follow up PET/CT scan showed an increase of the left lung mass size, 1.3X1.5X1.8 cm, however, a slight decrease in SUV, 1.3, was also observed (Figure 4, top panel).

### DETAILED DESCRIPTION

An "agent," "drug" or "therapeutic agent" refers to a chemical compound, a mixture of chemical compounds, a biological macromolecule, or an extract made from biological materials such as bacteria, plants, fungi, or 60 animal (particularly mammalian) cells or tissues that are suspected of having therapeutic properties. The agent or drug may be purified, substantially purified or partially purified. Examples of agents may include, but are not limited to, chemotherapeutic agents, targeted cancer therapies (e.g., therapeutic antibodies or functional fragments thereof, and immunologic agents, therapeutic antibodies. An "agent", according to the present invention, also includes a radiation therapy agent.

"Antibody or functional fragment thereof" means an immunoglobulin molecule that specifically binds to, or is immunologically reactive with a particular antigen or epitope, and includes both polyclonal and monoclonal antibodies. The term antibody includes genetically engineered or otherwise modified forms of immunoglobulins, such as intrabodies, peptibodies, chimeric antibodies, fully human antibodies, humanized antibodies, and heteroconjugate antibodies (e.g., bispecific antibodies, diabodies, triabodies, and tetrabodies). The term functional antibody fragment includes antigen binding fragments of antibodies, including e.g., Fab', F(ab')₂, Fab, Fv, rlgG, and scFv fragments. The term scFv refers to a single chain Fv antibody in which the variable domains of the heavy chain and of the light chain of a traditional two chain antibody have been joined to form one chain.

A "chemotherapeutic agent" is any agent used to treat cancer. Chemotherapeutic agents have many mechanisms of action, some of which are non-specific, affecting all cells in the body, while others are specific or targeted to cancer cells. The term chemotherapeutic agent includes all antineoplastic drugs, including small molecules, biologics, immunologic agents, targeted therapies, cytotoxic or cytolytic agents, alkylating agents, antimetabolites, anti-tumor antibiotics, topoisomerase inhibitors, or any other agent that is used to kill cancer cells, slow or stop cancer cell division, slow or stop cancer cell metastasis or otherwise treat cancer.

An "immunologic agent," "immunotherapeutic" or "immunotherapeutic agent" is a substance or treatment having active or passive immunostimulant activity. Such activity may be a result of specific immunostimulants or non-specific immunostimulants. In addition to the above definition, the term "immunotherapy" includes behaviors or treatments that may directly or indirectly cause an increase in an immune response or causes an increase in an immune response relative to or in comparison to other therapies.

The term "immunostimulant" encompasses all substances, treatments or behaviors which influence the function of cells which are involved directly or indirectly in mediation of the immune response, and where the influence leads to an immune response. These cells include, for example, macrophages, natural killer cells, Langerhans cells and other dendritic cells, lymphocytes, indeterminate cells, fibroblasts, keratinocytes and melanocytes.

"In combination" or "in combination with," as used herein, means in the course of treating the same disease in the same patient using two or more agents, drugs, treatment regimens, treatment modalities or a combination thereof, in any order. This includes simultaneous administration, as well as in a temporally spaced order of up to several days apart. Such combination treatment may also include more than a single administration of any one or more of the agents, drugs, treatment regimens or treatment modalities. Further, the administration of the two or more agents, drugs, treatment regimens, treatment modalities or a combination thereof may be by the same or different routes of administration. In the embodiments described herein, one or more chemotherapeutic agents may be administered, alone or in combination, to a subject for curative or palliative treatment of cancer.

A "pharmaceutically acceptable carrier" refers to a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluent, excipient, solvent, or encapsulating material, or some combination thereof. Each component of the carrier must be "pharmaceutically acceptable" in that it must be compatible with the other ingredients of the formulation. It also must be suitable for contact with any tissue, organ, or portion of the body that it may encounter, meaning that it must not carry a risk of toxicity, irritation, allergic response, immunogenicity, or any other complication that excessively outweighs its therapeutic benefits.

"Route of administration" may refer to any administration pathway known in the art, including but not limited to aerosol, enteral, nasal, ophthalmic, oral, parenteral, rectal, transdermal (e.g., topical cream or ointment, patch), or vaginal. "Parenteral" refers to a route of administration that is generally associated with injection, including infraorbital, infusion, intraarterial, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal.

"Targeted cancer therapies," "molecularly targeted drugs," or "molecular targeted therapies" are drugs or other substances that block the growth and spread of cancer by interfering with specific molecular targets that are involved in tumor growth and progression. Although targeted cancer therapeutics may be considered as a type of chemotherapy, they are often considered a separate group. Targeted cancer therapies are typically a small molecule drug or a therapeutic antibody or functional fragment thereof.

"Treating" or "treatment" of a condition such as cancer may refer to preventing the condition, slowing the onset or rate of development of the condition, reducing the risk of developing the condition, preventing or delaying the development of symptoms associated with the condition, reducing or ending symptoms associated with the condition, generating a complete or partial regression of the condition, or any combination thereof.

A "standard dose" of a particular cancer treatment, including chemotherapeutics and targeted cancer therapies, is typically a maximum safe dosage. A "maximum safe dosage" or "maximum recommended therapeutic dosage" is the highest amount of a therapeutic agent that can be given that minimizes complications or side effects to a patient while maintaining its efficacy as a treatment. Such a dose can be adjusted to consider the patient's overall heath and any extenuating factors that could hamper the patient's recovery. Due to the severity and potential lethal outcome of the disease, a maximum safe dosage tolerated in cancer treatment may be an amount that causes considerable and severe side effects.

A "therapeutically effective amount," "effective amount" or "effective dose" is an amount of a therapeutic agent that produces a desired therapeutic effect in a subject, such as preventing or treating a target condition or alleviating symptoms associated with the condition. The precise therapeutically effective amount is an amount of the composition that will yield the most effective results in terms of efficacy of treatment in a given subject. This amount will vary depending upon a variety of factors, including but not limited to the characteristics of the therapeutic compound (including activity, pharmacokinetics, pharmacodynamics, and bioavailability), the physiological condition of the subject (including age, sex, disease type and stage, general physical condition, responsiveness to a given dosage, and type of medication), the nature of the pharmaceutically acceptable carrier or carriers in the formulation, and the route of administration. One skilled in the clinical and pharmacological arts will be able to determine a therapeutically effective amount through routine experimentation, namely by monitoring a subject's response to administration of a compound and adjusting the dosage accordingly. For additional guidance, see Remington: The Science and Practice of Pharmacy 21st Edition, Univ. of Sciences in Philadelphia (USIP), Lippincott Williams & Wilkins, Philadelphia, PA, 2005

In some embodiments, a therapeutically effective dose of a particular agent may be the same as or lower than a standard dose. In a preferred embodiment, the therapeutically effective dose is lower than a standard dose. A therapeutically effective dose for a particular agent used in accordance with the embodiments described herein may be a dose that is a fraction or a percentage of a standard dose for that particular agent. In some aspects, a therapeutically effective dose may be between about 1% and 99% of a standard dose, between about 1% and 90% of a standard dose, between about 1% and 80% of a standard dose, between about 1% and 70% of a standard dose, between about 1% and 60% of a standard dose, between about 1% and 50% of a standard dose, between about 1% and 40% of a standard dose, between about 1% and 30% of a standard dose, between about 1% and 20% of a standard dose, between about 5% and 20% of a standard dose, between about 1% and 10% of a standard dose or below about 10% of a standard dose for a particular agent. In one aspect, a therapeutically effective dose of a particular agent may be about 10% of a standard dose, about 1% of a standard dose, or lower than 1% of a standard dose for a particular agent. In yet another aspect, a therapeutically effective dose may be between about 0.1% and 1% of a standard dose, between about between about 0.01% and 1% of a standard dose or between about 0.001 % and 1% of a standard dose for a particular agent.

Provided herein is a metabolic targeting chemo-immunotherapy regimen for use in the treatment of cancer, the metabolic targeting chemo-immunotherapy regimen comprising:
cetuximab at a dose of 20 mg;
insulin at a dose of 0.1 to 0.5 units/kg to reduce blood glucose level to 2.2 to 2.8 mmol/L; and
a therapeutically effective dose of one or more chemotherapeutic agents, to be administered at a blood glucose level of 2.2 to 2.8 mmol/L.

The regimen of metabolic targeting chemo-immunotherapy includes a metabolic targeting chemotherapy treatment regimen used in combination with an immunologic targeting treatment regimen, both of which are described further below. In one embodiment of the disclosure, the regimen includes administering a therapeutic antibody or functional fragment thereof in combination with administration of one or more chemotherapeutic agents under low blood glucose conditions.

The metabolic targeting chemo-immunotherapy regimens described herein may be used to treat any cancer or tumor type. Cancers and tumor types that may be treated using the metabolic targeting chemo-immunotherapy regimens described herein include but are not limited to bone cancer, bladder cancer, brain cancer, breast cancer, cancer of the urinary tract, carcinoma, cervical cancer, colon cancer, esophageal cancer, gastric cancer, head and neck cancer, hepatocellular cancer, liver cancer, lung cancer, lymphoma and leukemia, melanoma, ovarian cancer, pancreatic cancer, pituitary cancer, prostate cancer, rectal cancer, renal cancer, sarcoma, testicular cancer, thyroid cancer, and uterine cancer. In addition, the regimens may be used to treat tumors that are malignant (*e*.*g*., cancers) or benign (*e.g.,* hyperplasia, cyst, pseudocyst, hamartoma, and benign neoplasm).

### Chemotherapy and other anti-cancer agents

Cancer treatment often involves chemotherapy alone or in combination with other modalities of treatments such as surgery, radiation therapy, targeted therapy and immunotherapy. Chemotherapy may be used to mean the use of any drug to treat any disease, but is often associated with cancer treatment. In the treatment of cancer, chemotherapeutics or chemotherapeutic agents are often referred to antineoplastic or anticancer agents. Many chemotherapeutic agents are cytotoxic or cytostatic in nature. Antineoplastic chemotherapeutic agents can be divided into several groups based on factors such as how they work, their chemical structure or source, and their relationship to another drug. Such groups include, but are not limited to, alkylating agents, antimetabolites, anti-tumor antibiotics, topoisomerase inhibitors, mitotic inhibitors hormone therapy, targeted therapeutics and immunotherapeutics. Some chemotherapeutics do not fit well into any categories. Because some drugs act in more than one way, they may belong to more than one group. Knowing how the drug works is important in predicting side effects.

Alkylating agents directly damage DNA to prevent the cancer cell from reproducing. These agents are not phase-specific, but instead work in all phases of the cell cycle. Alkylating agents are used to treat many different cancers, including acute and chronic leukemia, lymphoma, Hodgkin disease, multiple myeloma, sarcoma, as well as cancers of the lung, breast, and ovary. Because these drugs damage DNA, they can cause long-term damage to the bone marrow. Alkylating agents that may be used according to the embodiments of the disclosure include, but are not limited to, nitrogen mustards (e.g., mechlorethamine (nitrogen mustard), chlorambucil, cyclophosphamide, ifosfamide and melphalan), nitrosoureas (e.g., streptozocin, carmustine (BCNU) and lomustine), alkyl sulfonates (e.g., busulfan), triazines (e.g., dacarbazine (DTIC) and temozolomide) and ethylenimines (e.g., thiotepa and altretamine (hexamethylmelamine)). In addition, the platinum drugs (cisplatin, carboplatin, and oxalaplatin) may be used according to the embodiments of the disclosure and are sometimes grouped with alkylating agents because they kill cells in a similar way.

Antimetabolites interfere with DNA and RNA growth by substituting for the normal building blocks of RNA and DNA. These agents damage cells during the S phase of the cell cycle. They are commonly used to treat leukemias, tumors of the breast, ovary, and the intestinal tract, as well as other cancers. Antimetabolites that may be used according to the embodiments of the disclosure include, but are not limited to, 5-fluorouracil (5-FU), capecitabine, 6-mercaptopurine (6-MP), methotrexate, gemcitabine, cytarabine, fludarabine and pemetrexed.

Anthracyclines are anti-tumor antibiotics that interfere with enzymes involved in DNA replication. These agents are not phase-specific. Thus, they are widely used for a variety of cancers. A major consideration when giving these drugs is that they can permanently damage the heart if given in high doses. For this reason, lifetime dose limits are often placed on these drugs. Anthracyclines that may be used according to the embodiments of the disclosure include, but are not limited to, daunorubicin, doxorubicin, epirubicin, and idarubicin. Other anti-tumor antibiotics include the drugs actinomycin-D, bleomycin, and mitomycin-C. In addition, mitoxantrone is another anti-tumor antibiotic that is similar to doxorubicin in many ways, including the potential for damaging the heart. This drug also acts as a topoisomerase II inhibitor (see below). Mitoxantrone is used to treat prostate cancer, breast cancer, lymphoma, and leukemia.

Topoisomerase inhibitors interfere with enzymes called topoisomerases, which help separate the strands of DNA so they can be copied. They are used to treat certain leukemias, as well as lung, ovarian, gastrointestinal, and other cancers. Topoisomerase inhibitors that may be used according to the embodiments of the disclosure include, but are not limited to, topoisomerase I inhibitors (e.g., topotecan and irinotecan (CPT-11) and topoisomerase II inhibitors (e.g., etoposide (VP-16), mitoxantrone and teniposide).

Mitotic inhibitors are often plant alkaloids and other compounds derived from natural products. They can stop mitosis or inhibit enzymes from making proteins needed for cell reproduction. These drugs generally work during the M phase of the cell cycle, but can damage cells in all phases. They are used to treat many different types of cancer including breast, lung, myelomas, lymphomas, and leukemias. Mitotic inhibitors that may be used according to the embodiments of the disclosure include, but are not limited to, taxanes (e.g., paclitaxel and docetaxel), epothilones (e.g., ixabepilone), vinca alkaloids (e.g., vinblastine, vincristine, and vinorelbine), and estramustine.

Some chemotherapy drugs do not fit well into any of the categories described above because they act in slightly different ways. Examples include, but are not limited to, L-asparaginase, which is an enzyme, and the proteosome inhibitor bortezomib.

Additional chemotherapeutics may include glycolysis inhibitors. As explained in more detail below, cancer cells are primarily glycolytic, relying heavily on the glycolysis pathway to generate ATP. The use of glycolysis inhibitors are thought to significantly reduce ATP generation, thereby preferentially killing cancer cells (Pelicano et al. 2006). However, glycolysis inhibitors can have toxic effects on healthy tissues, such as the brain, that also rely on glycolysis for energy. Thus, as with other types of chemotherapeutics, lowering blood glucose levels would allow glycolysis inhibitors to be used at lower doses to sensitize and more effectively target cancer cells. Glycolysis inhibitors target components of the glycolytic pathway such as hexokinase (HK), glugose-6-phosphate dehydrogenase (G6PG), transketolase-like enzyme 1 (TKTL1), glyceraldehyde-3-phosphate dehydrogenase (GAPDH) and pyruvate dehydrogenase kinase (PDK). In addition, glycolysis inhibitors may be non-metabolizable glucose analogs. Examples of glycolysis inhibitors that may be used in accordance with the embodiments described herein include, but are not limited to, α-chlorohydrin, 6-aminonicotinamide (6-AN), arsenic compounds, 3-BrOP, 3-bromopyruvate (3-PrPA), bromopyruvic acid, 2-deoxy-D-glucose (2-DG), dichloroacetic acid, dichloroacetates and related salts, genistein, glufosfamide, imatinib, lonidamine, mannoheptulose, ornidazole, oxalate, oxythiamine, SB-204990, and 5-thioglucose.

Targeted cancer therapies block the growth and spread of cancer by interfering with specific molecules involved in tumor growth and progression. Targeted therapies may have properties or characteristics of more than one category of chemotherapeutic agents, including cytotoxic agents, hormone therapy, biologic therapy and immunotherapy. For example, therapeutic antibodies are biologic agents that have chemotherapeutic and immunotherapeutic characteristics, as described further below. In addition, although targeted therapies often provide an efficient method for tailoring cancer treatment based on the type of cancer, and/or the unique set of molecular targets produced by a patient's tumor, they have several limitations including side effects (e.g., allergic reactions, chills, fatigue, fever, muscle aches and pains, nausea, diarrhea, skin rashes, heart failure, skin infections and bleeding) and the potential for developing resistance to these therapeutics. In many cases, once resistance occurs, alternative targeted therapies do not exist.

Examples of targeted therapies that may be used in accordance with any of the treatment regimens described herein include, but are not limited to, selective estrogen receptor modulators (SERMs) (e.g., tamoxifen, toremifene and fulvestrant), aromatase inhibitors (anastrozole, exemestane and letrozole, kinase inhibitors (imatinib mesulate, dasatinib, nilotinib, lapatinib, gefitinib, erlotinib, temsirolimus and everolimus, growth factor receptor inhibitors (e.g., Trastuzumab, cetuximab and panitumumab), regulators of gene expression (vorinostat, romidepsin, bexarotene, alitretinoin and tretinoin), apoptosis inducers (bortezomib and pralatrezate), angiogenesis inhibitors (bevacizumab, sorafenib, sunitinib and pazopanib), antibodies that triggers a specific immune response by binding a cell-surface protein on lymphocytes (rituximab, alemtuzumab and ofatumumab), antibodies or other molecules that deliver toxic molecules specifically to cancer cells (tositumomab, ibritumomab tiuxetan, denileukin diftitox), cancer vaccines and gene therapy.

In some embodiments, chemotherapeutic agents, used alone or in combination, that may be used to treat cancer according to the embodiments described herein may include, but are not limited to, 13-cis-Retinoic Acid, 2-Chlorodeoxyadenosine, 5-Azacitidine, 5-Fluorouracil, 6-Mercaptopurine, 6-Thioguanine, actinomycin-D, adriamycin, aldesleukin, alemtuzumab, alitretinoin, all-transretinoic acid, alpha interferon, altretamine, amethopterin, amifostine, anagrelide, anastrozole, arabinosylcytosine, arsenic trioxide, amsacrine, aminocamptothecin, aminoglutethimide, asparaginase, azacytidine, bacillus calmette-guerin (BCG), bendamustine, bevacizumab, bexarotene, bicalutamide, bortezomib, bleomycin, busulfan, calcium leucovorin, citrovorum factor, capecitabine, canertinib, carboplatin, carmustine, cetuximab, chlorambucil, cisplatin, cladribine, cortisone, cyclophosphamide, cytarabine, darbepoetin alfa, dasatinib, daunomycin, decitabine, denileukin diftitox, dexamethasone, dexasone, dexrazoxane, dactinomycin, daunorubicin, decarbazine, docetaxel, doxorubicin, doxifluridine, eniluracil, epirubicin, epoetin alfa, erlotinib, everolimus, exemestane, estramustine, etoposide, filgrastim, fluoxymesterone, fulvestrant, flavopiridol, floxuridine, fludarabine, fluorouracil, flutamide, gefitinib, gemcitabine, gemtuzumab ozogamicin, goserelin, granulocyte - colony stimulating factor, granulocyte macrophage-colony stimulating factor, hexamethylmelamine, hydrocortisone hydroxyurea, ibritumomab, interferon alpha, interleukin-2, interleukin-4, interleukin-11, isotretinoin, ixabepilone, idarubicin, imatinib mesylate, ifosfamide, irinotecan, lapatinib, lenalidomide, letrozole, leucovorin, leuprolide, liposomal Ara-C, lomustine, mechlorethamine, megestrol, melphalan, mercaptopurine, mesna, methotrexate, methylprednisolone, mitomycin C, mitotane, mitoxantrone, nelarabine, nilutamide, octreotide, oprelvekin, oxaliplatin, paclitaxel, pamidronate, pemetrexed, panitumumab, PEG Interferon, pegaspargase, pegfilgrastim, PEG-L-asparaginase, pentostatin, plicamycin, prednisolone, prednisone, procarbazine, raloxifene, rituximab, romiplostim, ralitrexed, sapacitabine, sargramostim, satraplatin, sorafenib, sunitinib, semustine, streptozocin, tamoxifen, tegafur, tegafur-uracil, temsirolimus, temozolamide, teniposide, thalidomide, thioguanine, thiotepa, topotecan, toremifene, tositumomab, trastuzumab, tretinoin, trimitrexate, alrubicin, vincristine, vinblastine, vindestine, vinorelbine, vorinostat, or zoledronic acid.

In other embodiments, the one or more chemotherapeutic agents used in the regimens described herein may correspond to known chemotherapeutic regimens known in the art including, but not limited to, ABVD, AC, BEACOPP, BEP, CA (or AC), CAF, CAV, CBV, ChIVPP/EVA, CHOP (or COHP), R-CHOP, COP (or CVP), CMF, COPP, EC, ECF, EP, EPOCH, FEC, FL (also known as Mayo), FOLFOX, FOLFIRI, ICE, ICE-R, m-BACOD, MACOP-B, MOPP, PCV, ProMACE-MOPP, ProMACE-CytaBOM, R-FCM, Stanford V, Thal/Dex, TIP, VAC, VAD, VAPEC-B, and VIP. Further explanation of these chemotherapeutic regimens is found in Table 1 below.

**Table 1. Known Chemotherapeutic Regimens.**

| **Regimen** | **Components** | **Example of uses, and other notes** |
|---|---|---|
| **ABVD** | Adriamycin (doxorubicin), bleomycin, vinblastine, dacarbazine | Hodgkin's lymphoma |
| **AC** | Adriamycin (doxorubicin), cyclophosphamide | Breast cancer |
| **BEACOPP** | Bleomycin, etoposide, Adriamycin (doxorubicin), cyclophosphamide, Oncovin (vincristine), procarbazine, prednisone | Hodgkin's lymphoma |
| **BEP** | Bleomycin, etoposide, platinum agent (cisplatin) | Testicular cancer, germ cell tumors |
| **CA** | Cyclophosphamide, Adriamycin (doxorubicin) (same as AC) | Breast cancer |
| **CAF** | Cyclophosphamide, Adriamycin (doxorubicin), fluorouracil (5-FU) | Breast cancer |
| **CAV** | Cyclophosphamide, Adriamycin (doxorubicin), vincristine | Lung cancer |
| **CBV** | Cyclophosphamide, BCNU (carmustine), VP-16 (etoposide) | Lymphoma |
| **ChIVPP/EV A** | Chlorambucil, vincristine (Oncovin), procarbazine, prednisone, etoposide, vinblastine, Adriamycin (doxorubicin) | Hodgkin's lymphoma |
| **CHOP or COHP** | Cyclophosphamide, hydroxydoxorubicin (doxorubicin), vincristine (Oncovin), prednisone | Non-Hodgkin lymphoma |
| **CHOP-R** or **R-CHOP** | CHOP + rituximab | B cell non-Hodgkin lymphoma |
| **COP** or **CVP** | Cyclophosphamide, Oncovin (vincristine), prednisone | Non-Hodgkin lymphoma in patients with history of cardiovascular disease |
| **CMF** | Cyclophosphamide, methotrexate, fluorouracil (5-FU) | Breast cancer |
| **COPP** | Cyclophosphamide, Oncovin (vincristine), procarbazine, prednisone | Non-Hodgkin lymphoma |
| **EC** | Epirubicin, cyclophosphamide | Breast cancer |
| **ECF** | Epirubicin, cisplatin, fluorouracil (5-FU) | Gastric cancer and oesophageal cancer |
| **EP** | Etoposide, platinum agent (cisplatin) | Testicular cancer, germ cell tumors |
| **EPOCH** | Etoposide, prednisone, Oncovin, cyclophosphamide, and hydroxydaunorubicin | Lymphomas |
| **FEC** | Fluorouracil (5-FU), epirubicin, cyclophosphamide | Breast cancer |
| **FL** (Also known as Mayo) | Fluorouracil (5-FU), leucovorin (folinic acid) | Colorectal cancer |
| **FOLFOX** | Fluorouracil (5-FU), leucovorin (folinic acid), oxaliplatin | Colorectal cancer |
| **FOLFIRI** | Fluorouracil (5-FU), leucovorin (folinic acid), irinotecan | Colorectal cancer |
| **ICE** | ifosfamide, carboplatin, etoposide (VP-16) | Aggressive lymphomas, progressive neuroblastoma |
| **ICE-R** | ICE + rituximab | High-risk progressive or recurrent lymphomas |
| **m-BACOD** | Methotrexate, bleomycin, Adriamycin (doxorubicin), cyclophosphamide, Oncovin (vincristine), dexamethasone | Non-Hodgkin lymphoma |
| **MACOP-B** | Methotrexate, leucovorin (folinic acid), Adriamycin (doxorubicin), cyclophosphamide, Oncovin (vincristine), prednisone, bleomycin | Non-Hodgkin lymphoma |
| **MOPP** | Mechlorethamine, Oncovin (vincristine), procarbazine, prednisone | Hodgkin's lymphoma |
| **PCV** | Procarbazine, CCNU (lomustine), vincristine | Brain tumors |
| **ProMACE-MOPP** | Methotrexate, Adriamycin (doxorubicin), cyclophosphamide, etoposide + MOPP | Non-Hodgkin lymphoma |
| **ProMACE-CytaBOM** | Prednisone, doxorubicin (adriamycin), cyclophosphamide, etoposide, cytarabine, bleomycin, Oncovin (vincristine), methotrexate, leucovorin | Non-Hodgkin lymphoma |
| **R-FCM** | Rituximab, fludarabine, cyclophosphamide, mitoxantrone | B cell non-Hodgkin lymphoma |
| **Stanford V** | Doxorubicin, mechlorethamine, bleomycin, vinblastine, vincristine, etoposide, prednisone | Hodgkin's lymphoma |
| **Thal/Dex** | Thalidomide, dexamethasone | Multiple myeloma |
| **TIP** | Paclitaxel, ifosfamide, platinum agent cisplatin | Testicular cancer, germ cell tumors in salvage therapy |
| **VAC** | Vincristine, Actinomycin, Cyclophosphamide | Rhabdomyosarcoma |
| **VAD** | Vincristine, Adriamycin (doxorubicin), dexamethasone | Multiple myeloma |
| **VAPEC-B** | Vincristine, Adriamycin (doxorubicin), prednisone, etoposide, cyclophosphamide, bleomycin | Hodgkin's lymphoma |
| **VIP** | Etoposide, ifosfamide, platinum agent cisplatin | Testicular cancer, germ cell tumors |

### Cell Fusion Leads to Carcinogenesis

The common dogma of how cancer forms is a series of genetic mutations which alters cells and leads to disease. Many studies have focused on chemical carcinogens and genetic instability resulting in mutations or aneuploidy of cancer-related genes. While these mutations are readily observed, they do not account for some traits of cancer, such as limitless replication/self renewal, differentiation into a heterogeneous population, and the ability to migrate through the body and survive in different tissue environments as in metastasis. These are the traits associated with what are described as cancer stem cells.

The discovery that many cancers arise from or contain stem cells that retain characteristics of normal stem cells (e.g., bone marrow derived stem cells, BMDSC) that allow them to survive for the lifespan of the individual. These characteristics include a low rate of cell division, active DNA repair and the expression of several transport proteins that protect cells against toxins, which makes the cancer stem cells relatively resistant to radiation and chemotherapy.

Cancer stem cells (CSCs) may arise from a normal stem cell that has undergone malignant transformation by accumulating genetic mutations or other abnormalities. Alternatively, cancer stem cells may arise from a stem cell fusion model of carcinogenesis that includes a fusion between a genetically altered cell and a stem cell of bone marrow origin. (He 2005). The stem cell fusion model of carcinogenesis is explained in detail in U.S. Patent Application Publication No. 20090016961, which is a national application of International Patent Application No. PCT/US06/033366, filed August 25, 2006. Stem cell fusion may represent a missing step in the understanding of carcinogenesis.

This "stem cell fusion model of carcinogenesis" provides insights on new strategies to target CSCs by targeting the common traits of bone marrow derived stem cells have with CSCs, namely their glycolysis based metabolism (i.e., Warburg effect, described further below). One additional trait of CSCs is their metabolism of glucose for energy.

Alterations in the AKT pathway is one of the most commonly seen transformation events in cancer. Additionally, high rates of glucose consumption (glycolysis) of cancer cells, commonly known as the Warburg effect, is correlated with a metastatic phenotype and a typical trait of cancer. The AKT pathway can regulate glycolysis through mTOR. Various inputs, such as insulin like growth factor (IGF-1) or hypoxia inducible factors send a single through the AKT pathway which activates mTOR, which in turn upregulates glycolysis enzymes (Elstrom 2004).

Tissue based hypoxia, which is a condition of low oxygen supply and often caused by a lack of blood flow, is commonly seen in poorly vascularized tumors. Cells in a hypoxic state use glycolysis for energy while mitochondrial respiration, which requires oxygen, is inhibited. Bone marrow stem cells survive in a hypoxic niche within marrow and obtain energy through anaerobic glycolysis (Simsek 2010). This process is regulated by hypoxia inducible factors (HIFs). CSCs express some of these HIFs and can also reside in a hypoxic niche within the tumor microenvironment. These cells use glucose for energy and maintain stem cell markers while in their hypoxic niche. (Heddlestin 2010).

Use of glucose for energy by cancer cells is retained even in the presence of oxygen. This aerobic glycolic trait, or Warburg effect, of cancer is often thought to be induced by mutations, especially in the AKT/mTOR pathway. However, the CSCs use of glycolysis for energy can in part be a result of epigenetic changes brought about by fusion with bone marrow derived stem cells, which also use glycolysis for their energy needs (Figure 1). The use of glycolysis maintains the stem cell phenotype which allows CSCs to self renew. This trait is inherited by the stem cell component of carcinogenic fusion. Thus, the metabolism of CSCs provides a unique target for therapy.

Evidence suggests that not only do cancer stem cells exist in solid tumors, but that they contribute to the invasive, malignant phenotype of these cancers. For example, a tumorigenic breast cancer stem cell population representing about 1% of the cells was found to be highly tumorigenic in mice, whereas the non-stem cells in the tumor were very poorly tumorigenic (Al-Hajj et al., 2003). Similarly, stem cells with a capacity to self-renew and undergo pluripotent differentiation have been isolated from human brain tumors and from lung tissue (Dean 2009).

### Cancer Stem Cells and Drug Transporters

Cancer is composed of a heterogeneous mix of cell populations. Although just a small component of the cell population, the cancer stem cells, are known to be able to recapitulate the entire heterogeneous population. This is best demonstrated by cancer regrowth after tumor debulking therapies such as surgery, chemotherapy and radiation. Many reports have demonstrated that CSCs are more resistant to chemotherapy agents than their non-stem cell counterparts. This is, in part, due to their ability to efflux drugs from the cell through use of multidrug resistance pumps, described in detail below.

This ability of CSCs is used in their isolation using Hoechst 33342 dye, in which the CSC population is able to exclude this fluorescent dye allowing the non-fluorescent CSC population to be selected. Targeting the CSC population has been of great interest and is currently being investigated in several clinical trials (Winquist 2009).

As discussed above, cancer stem cells retain characteristics of normal stem cells. One such characteristic is a high expression levels of specific ATP-dependent ABC drug transporter (or multi-drug resistance (MDR) pump) genes, including, but not limited to, ABCB1 (which encodes the P-glycoprotein transporter), ABCC1 (which encodes the MRP1 transporter), ABCC2 (which encodes the MRP2 transporter), ABCG2 (which encodes the breast cancer resistance protein, BCRP), ABCA2 (which encodes ABC2), and ABCB11 (which encodes the "sister of P-glycoprotein," SPGP) (Leonard et al. 2003). These genes are members of the ATP-binding cassette (ABC) transporter superfamily and represent the major tumor multi-drug resistance genes. Expression of these MDR pumps allows cells to pump drugs out, conferring resistance to chemotherapeutics including, but not limited to, adramycin, daunorubicin, epirubicin, paclitaxel, docetaxel, vincristine, vinblastine, VP-16, mitoxantrone, actinomycin-D, doxorubicin, topoisomerase I or II inhibitors and anthracyclines (Leonard et al. 2003). Tumors that recur after an initial response to chemotherapy are often multi-drug resistant (Gottesman et al., 2002).

The drug transporting property of stem cells is an important phenotype for the isolation of hematopoietic stem cells. Stem cells exclude fluorescent dyes because the dyes are removed by ABCG2 and ABCB1. Therefore, stem cells can be sorted by collecting the cells that contain only a low level of fluorescence referred to as the "side population" (SP cells, SP phenotype). Because stem cells are predominantly found in the SP fraction, it is possible to sort and purify stem cells from virtually any population of cells or tissue, including cancer. SP cells were identified in 15 out of 23 neuroblastoma samples and in neuroblastoma, breast cancer, lung cancer, and glioblastoma cell lines. Furthermore, analysis of several cell lines demonstrated a small population of SP cells. Therefore, even long-established tumor cell lines contain a cancer stem cell population, strongly supporting the idea that this is a fundamental property of cancers.

In a tumor stem cell paradigm, the cancer stem cells are naturally resistant to chemotherapy through their quiescence, their capacity for DNA repair, and ABC transporter expression. As a result, at least some of the tumor stem cells survive chemotherapy and support re-growth of the tumor. The resistance phenotype of the cancer stem cell persists in the committed, abnormally developing progenitors that comprise the recurrent tumor. Therefore, cancer stem cells may account for recurrence of cancer after treatment by surviving traditional cancer therapies (Dean 2009).

By inhibiting chemotherapy drug transporters, resistance may be overcome allowing complete elimination of the tumor. Therefore, ABC transporter inhibitors may be used to target cancer stem cells expressing drug transporters that make them resistant to many chemotherapy agents. ABCB1 inhibitors have shown limited effectiveness in clinical trials, however, these studies have not focused on targeting cancer stem cells (Dean 2009).

### Metabolic Targeting Chemotherapy Treatment

Due to their dependence on glycolysis for energy, CSCs can be killed directly or indirectly by targeting glycolysis, either through antagonists of glycolysis metabolism and/or limiting the availability of glucose. Additionally, CSCs drug resistance can be overcome by limiting glycolysis through mechanisms which inhibit ATP production necessary to run ABC transporters, as previously described.

A negative regulator of the AKT/mTOR pathway is AMP-activated protein kinase (AMPK), which is activated in response to an increased ratio of AMP to ATP. An example of this function would be when low blood glucose levels limit ATP produced by glycolysis leading to an increase in AMP. The AMPK "energy sensor" directs increased consumption of fatty acids metabolism through mitochondria respiration and a decrease in glycolysis. In the context of CSCs, modulating this pathway to decrease glycolysis can deprive the CSCs of necessary ATP (Xu 2005).

Metformin is derived from French lilac, which has been used for centuries to treat symptoms of diabetes mellitus. Metformin belongs to a class of drugs called biguanides, which acts in an indirect manner on AMPK, which in turns suppresses the AKT/mTOR pathway. In the diabetic setting, metformin suppresses hepatic gluconeogenesis, the liver's ability to make glucose and thereby lower blood glucose levels.

People with diabetes have an increased risk of dying from cancer. However, patients taking metformin have a reduced cancer risk and a lower cancer related mortality. In an epidemiological study of 2,529 women with breast cancer, diabetic women taking metformin had a higher pathological complete response (pCR) rate to neoadjuvant systematic therapy compared to both diabetic and non-diabetic woman not taking metformin (diabetic metformin group - 24% pCR, diabetic control - 8% pCR, non-diabetic control - 16%) (Dowling 2011; Hirsch 2009). Due to metformin's inhibition of the AKT/mTOR pathway via AMPK, glycolysis of CSCs can be impacted in a negative fashion leading to increased susceptibility of drugs and cell death.

Most chemotherapy agents act directly or indirectly on DNA. DNA is most vulnerable to damage while unwound from chromatin and histones to undergo DNA replication during S phase. Cancer cells are known for their uncontrolled cell growth. However, normal cells are growth restricted based on nutrient availability and can sense overall energy levels and postpone cell division. Under fasting conditions and/or hypoglycemia and/or modulation of metabolic pathways, normal cells stop the cell division process and go into a G1 cell cycle block.

Mutations in growth pathways common in cancer cells can inhibit this response. This leads to a "differential stress response" where normal cells are protected from the effects of chemotherapies while dividing cancer cells are left vulnerable (Raffaghello 2008). Normal cells and the tissues they make up are more resistant to chemotherapy under hypoglycemic or metabolically shifted conditions leading to fewer side effects and allowing for more frequent or higher dose treatments.

Therefore, in some embodiments, the one or more chemotherapeutic agents used in the regimens described herein may include a plurality of chemotherapeutic agents, each of which target a different point in the cell cycle. This results in targeting a higher percentage of cancer cells or cancer stem cells with each dose.

Standard chemotherapy is generally administered once every 3 weeks. These drugs are often administered intravenously over several hours. Since cancer cells are their most vulnerable to many chemotherapeutic drugs during S phase, the frequency of chemotherapy treatments (i.e., how often it is administered) is important to the success of therapy.

Cancer cells are killed in a dose-dependant manner with increasing dose leading to more cell death. Dose dense regimens, or high frequency regimens, where chemotherapy is administered more often and at a higher overall dose, have been used in the past with mixed results but higher toxicity (Bonilla 2010). Continued administration of these treatments is inhibited by the higher toxicity and the efficacy is limited by the CSCs resistance to therapy.

The strategy for treatment with chemotherapy described herein greatly relieves many of the side effects experienced with standard therapy and as such, side effects are not a constraint to frequent therapy. Cancer cells undergo their vulnerable S phase frequently, but not frequent enough for most of them to be affected by standard chemotherapy schedules. Alternative mitotic blockers, a class of drugs which target microtubules, are only effective during M2 phase of cell division.

Different chemotherapeutic drugs target different parts of the cell cycle. Because the time window during which the blood glucose level is lowered (minutes) is too short as compared to the duration of a cancer cell's cell cycle (hours or days), only a small fraction of cancer cells will be targeted and/or affected per treatment.

The cell cycle "window of opportunity" occurs for most of the cancer cell population outside of the pharmacological activity of the administered drugs. Even dose dense therapies are often only given weekly, leaving many cancer cells in a protected cell cycle phase during treatment. However, killing of cancer cells, and in particular, killing of cancer stem cells, may be enhanced by increasing the frequency of the treatments. To maximize the therapeutic efficacy, one should treat with a high frequency (i.e., as many times as possible) to target different parts of cell cycle. Thus, in some embodiments, the frequency of administering a chemotherapeutic agent is a high frequency and is selected from daily, every other day, 3 times weekly or biweekly.

In some embodiments, the more frequent or high frequency therapy is combined with conditions which not only sensitize cancer cells and CSCs to chemotherapy but also under conditions which protects normal cells. Delivering more frequent chemotherapy under hypoglycemic/metabolically modulated conditions will yield fewer side effects while increases efficacy by damaging cells in vulnerable cell cycles. Additionally, affecting glycolysis will increase sensitivity of CSCs to treatment by limiting ATP needed to run ABC transporters.

In some embodiments, the treatment regimen disclosed herein includes administering a therapeutically effective amount of one or more chemotherapeutic agents that is lower than a standard dose in combination with a method for lowering a subject's blood glucose, I.e. Insulin administration, to sensitize the cancer cells to the one or more chemotherapeutic agents. Sensitization of cancer cells to a chemotherapeutic agent in response to a decrease in blood sugar, renders the cancer cells more sensitive to the effects of the chemotherapeutic agent as compared to healthy cells (i.e., low blood sugar potentiates a chemotherapeutic agent's effect in cancer cells). Potentiation of a chemotherapeutic agent's effect results in an effective amount of chemotherapy to be lower than a standard dose, thereby reducing the side effects caused due to damage or death to healthy cells.

In some embodiments, the treatment regimen for treating cancer as disclosed herein may include a combination therapy, which affects multiple aspects of metabolism and provides synergistic efficacy not seen with individual compounds or treatments. The combination therapy includes administering an effective amount insulin to lower blood glucose and also administering an effective amount of metformin, both of which will inhibit glycolysis by limiting the supply of glucose and inhibiting pro-glycolysis signaling, respectively. In some embodiments, the administration of metformin may be continued after the administration of the chemotherapeutic agent is stopped or finished. Further, in one embodiment, the treatment regimens described herein may include administration of insulin or an insulin-dependent agent to induce hypoglycemia, followed by administration of metformin concurrently with one or more chemotherapeutic agents. The administration may be continued after the administration of the one or more chemotherapeutic agents has been stopped.

This is turn will reduce available ATP, necessary for the function of ABC transports which actively remove drug for CSCs. Additionally, limiting side effects of chemotherapy drugs by lowering blood glucose during treatment and using lower doses of the drugs allows for a treatment using immune system modulators to work more efficiently and allows for more frequent treatments.

The treatment regimens disclosed herein include administering a therapeutically effective dose of one or more chemotherapeutic agents to a subject having cancer after lowering said subject's blood glucose level. The chemotherapeutic agent may be, but is not limited to, any of the agents or combination regimens described above or a combination thereof.

Lowering of a subject's blood glucose in accordance with the embodiments of the disclosure is accomplished by insulin administration at a dose of 0.1 to 0.5 units/kg to reduce blood glucose level to 2.2 to 2.8 mmol/L.

Lowering of the subject's blood glucose level is accomplished by administering a dose of insulin sufficient to reduce blood glucose levels, the dose of insulin being approximately 0.1 to 0.5 units/kg. In one embodiment, the dose of insulin is approximately 0.2 units/kg. The dose of insulin is sufficient to reduce blood glucose level to roughly half of a baseline or fasting blood glucose level, to about 2.2-2.8 mmol/L. Reducing blood sugar may potentiate the effect of chemotherapeutic agents through several mechanisms. Lowering blood glucose exploits the reliance on glucose by cancer cells to produce energy, also known as the Warburg effect (Warburg 1925). Cancer cells are glycolytic, and must consume glucose for their energy needs. The glucose is consumed anaerobically by cancer cells, yielding less energy than aerobic respiration. As a consequence, cancer cells must consume a large amount of glucose. Because of this effect, any method to lower blood glucose in a cancer patient may put cancer cells in a starvation state, making the cancer cells more sensitive to chemotherapeutic agents.

As discussed above, cancer stem cells retain many characteristics of normal stems cells, including chemoresistance characteristics as a result of increased expression of multi-drug resistance (MDR) pumps (Leonard et al. 2003). Because MDR pumps need ATP to work, lowering blood glucose levels reduces the available ATP available to the cancer stem cells to pump the chemotherapeutic drugs out of the cell, thereby disrupting the MDR pumps and overcoming the multi-drug resistance often seen in tumors. Further, targeting of MDR pumps in cancer stem cells by reducing blood glucose levels may be enhanced by administering a therapeutically effective dose of one or more MDR pump inhibitors in combination with a reduction in blood glucose.

In addition, the use of insulin for lowering blood sugar may further potentiate the effect of chemotherapeutic agents in cancer cells. Cancer cells from many types of cancer have been observed to have more insulin receptors than normal cells (Ayre et al. 2000; Abita et al. 1984). Therefore, the use of insulin to decrease blood glucose levels is thought to increase permeability of cancer cells to a greater extent than in normal cells, making the cancer cells more vulnerable to chemotherapeutic agents, increasing their efficacy. (Ayre et al., 2000). For example, a 2-fold increase in the uptake of elipticine by MDA-MB-231 breast cancer cells has been observed when the cells were incubated with insulin (Oster et al. 1981). Another study showed a 50% stimulation of uptake of methotrexate by breast cancer cells when the cells were incubated with insulin (Shilsky et al. 1971).

Further, in some embodiments, the reduction in blood sugar is maintained for a prolonged period of time, i.e., the reduction is maintained for as long as safely practicable. The longer one can safely keep the blood sugar down, the more favorable result will occur.

This increase in cancer cell permeability and resulting increased uptake of chemotherapeutic agents may be due to an increase in insulin receptor or IGF-1 receptor-mediated transport (Poznansky et al. 1984; Yoshimasa et al. 1984; Gasparro et al. 1986; Ayre 1989), but may also be due to alterations in cellular lipid synthesis causing an increase in membrane fluidity (Jeffcoat & Jame 1984; Shinitzky et al. 1971; Jeffcoat 1979).

Insulin can also stimulate division of cancer cells, increasing the S-phase fraction in tumors, rendering the cells more susceptible to the cytotoxic effects of chemotherapeutic agents. Many chemotherapeutics act by targeting rapidly dividing cells as discussed above. Cancer cells are rapidly dividing cells, but only some cells are actively growing at any time, which means you can only kill some of the malignant cells at any time with conventional chemotherapy. Because insulin stimulates division in cancer cells, a higher percentage of the cancer cells divide at the same time, enabling chemotherapeutics to be absorbed by a much higher percentage of cancer cells. One study has shown that the addition of insulin to an asynchronous population of breast cancer cells increased the S-phase fraction to 66%, compared to only 37% in controls (Gross et al. 1984). Given the pharmacokinetics of neoplastic agents, particularly the cell-cycle phase specific agents, such an increase in the S-phase fraction would likely have a significant effect to enhance anticancer drug cytotoxicity.

*In vitro* data has shown the potentiation of chemotherapeutic agents in response to insulin. For example, when the chemotherapeutic agent methotrexate is administered with insulin to breast cancer cells in culture, the same percent cell killing is achieved with concentrations of methotrexate that are 10⁴ lower than when methotrexate is administered alone (Alabaster et al. 1981).

Another receptor often found in greater numbers in cancer cells than in normal cells of the same tissue type is the insulin-type growth factor-1 receptor (IGF-1 receptor or IGF-1 R). IGF-1 is a peptide of 70 amino acid residues having 40% identity with proinsulin. Insulin and IGF-1 have some cross-reactivity with each other's receptor. Therefore, in some embodiments, IGF-1 or any suitable IGF-1 receptor agonist may be administered in addition to or as an alternative to insulin and may have the same or similar effect as insulin.

### Fluorine-18 Fluorodeoxyglucose Positron Emission Tomography (FDG-PET) Use in Metabolic Targeted Chemo-Immunotherapy

FDG-PET is a commonly used scanning technology for oncology based on positron emitting isotope radiation. Combined with x-ray based computed tomography (CT), PET/CT is capable of three dimensional anatomical imaging overlaid with biochemical based scanning. Based on the Warburg effect, FDG-PET is able to detect cancers which uptake glucose more readily than surrounding tissue. This ratio between normal tissue glucose uptake and cancer cell glucose uptake is calculated into a Standard Uptake Value (SUV) where a high value SUV correlates with a high glucose metabolism.

An FDG-PET scan presenting a lesion with a high SUV is indicative of malignancy of various cancers and is useful in staging of cancers. Traditionally, while an initial high SUV is predictive of malignancy and able to find metastasis, it not predictive of treatment outcome. A second FDG-PET scan post treatment with traditional chemo-radiation therapies is usually necessary to predict efficacy of treatment. (Workman 2006) (Weber 2005)

Because the Metabolic Targeted Chemo-Immunotherapy described herein specifically targets cancer cells and CSCs, which use high levels of glucose, initial FDG-PET scanning is predictive of our treatment outcome. Lesions with relative high SUV values, which can vary between different machines and sites performing the scan but generally greater than 2.5 SUV, will be susceptible to our treatment. These high glucose using lesions will respond well to Metabolic Targeted Chemo-Immunotherapy causing regression in those lesions.

In some embodiments, the regimens of metabolic targeted chemo-immunotherapy described herein preferentially targets a population of cancer cells over healthy cells. The population of cancer cells may be a population of cancer stem cells having a high standard uptake value (SUV).

### Synergy between Metabolic targeting Chemotherapy and Immunologics.

The idea that malignant cancer cells retain stem cell characteristics because they come from fusion between stem cells and non-malignant cells suggests it is also possible to turn malignant cancer cells into antigen-presenting cells (APCs) which makes them detectable by the immune system (Figure 2).

Treatment of mesenchymal stem cells (MSCs) with interferon-Gamma causes them to become APCs (Chan et al). Other APCs (ie. Dendritic cells) can be derived by treating monocytes progenitor cells with GM-CSF, IL-4 and TNF-alpha (Ohgimoto et al.). Therefore, according to some embodiments, the regimens described herein may include treating malignant cancer cells with interferon-Gamma, causing them to become APCs' and treating malignant cancer cells with GM-CSF, IL-4 and TNF-alpha, causing the cells to turn into dendritic cell-like APCs.

### Immunotherapy and Immunologic Targeting Treatment

The regimen disclosed herein for treating cancer includes an immunologic targeting treatment regimen. The immunologic targeting treatment regimen includes cetuximab at a dose of 20 mg.

In some embodiments, metabolic targeting chemotherapeutic treatments as described above may, in effect, be considered an immunotherapy due to its immune cell sparing effect. By reducing the damage to the immune system and its innate ability to target cancer cells, an immunotherapeutic effect is achieved when compared to typical chemotherapy or combination chemotherapy.

Immunologic agents can have direct cytotoxic and/or immunological effects against tumor cells. They are often administered in conjunction with chemotherapy in treatment of cancers. However, standard doses of cancer chemotherapy have significant immune toxicity. This makes the therapeutic efficacy of chemotherapy and immunologics difficult to combine.

As described above, a metabolic targeting chemotherapy regimen may include reducing a patient's blood glucose level and administering a therapeutically effective dose of one or more chemotherapeutic agents, wherein the therapeutically effective dose is lower than a standard dose of chemotherapy. By using chemotherapy drugs at lower than standard doses, the cells of the immune system will be, in large part, spared. Thus, an immune response against opportunistic infections and the cancer itself may be more effectively induced by administering an immunologic targeting treatment regimen in combination with the metabolic targeting chemotherapy regimen. The combination of low-dose chemotherapy with immunotherapy will result in a synergistic therapeutic effect.

Therefore, the regimens for treating cancer disclosed herein include administering a metabolic targeting chemotherapy treatment regimen in combination with an immunologic targeting treatment regimen. The immunologic targeting treatment regimen includes administering cetuximab at a dose of 20 mg to stimulate an immune response in a subject having cancer.

There are two main types of immunologic agents, active and passive. Active immunologic agents, such as vaccines, stimulate an immune response to one or more specific antigenic types. In contrast, passive immunologic agents do not have antigenic specificity but can act as general stimulants that enhance the function of certain types of immune cells. Immunologic agents that may be used in an immunologic targeting treatment regimen may include immunostimulant substances that modulate the immune system by stimulating the function of one or more of the system's components. The regimens described herein may be combined with behaviors or treatments that may stimulate the immune system including, but not limited to, exercise, meditation, yoga, deep breathing, tai chi/Qigong, and acupuncture.

The cetuximab used in the regimens described herein may be a functional fragment of said antibody that targets cancer cells. Passive immunotherapy in the form of therapeutic antibodies has been the subject of considerable research and development as anti-cancer agents. Therapeutic antibodies are typically administered in maximum tolerated doses to block target receptors that are overexpressed on cancer cells, blocking the receptor's function systemically. However, given at a dose that is substantially lower than the maximum tolerated dose (e.g., ½ to 1/1000^{th} of the standard dose, as disclosed herein) allows the therapeutic antibody to act as an immunostimulant. After binding a target cancer cell, therapeutic antibodies or functional fragments thereof may stimulate cytotoxic immune-mediated responses, such as antibody-dependent cell-mediated cytotoxicity and complement-dependent cytotoxicity, mediated by Fc region activation of complement or Fc receptor (FcR) engagement. After cancer cells have been lysed, macrophages and other phagocytic, antigen presenting immune cells may engulf the components of the lysed cell and present cancer cell antigens to stimulate an acquired immune response against the cancer cells.

Examples of therapeutic antibodies that may be used as an immunologic agent include, but are not limited to, alemtuzumab, bevacizumab, cetuximab, edrecolomab, gemtuzumab, ibritumomab tiuxetan, panitumumab, rituximab, tositumomab, and trastuzumab.

The therapeutic agent and/or chemotherapeutic agent used in the treatment regimens described herein may be administered to a patient as part of a pharmaceutical composition or formulation. In some embodiments, the pharmaceutical composition or formulation may include cetuximab, one or more chemotherapeutic agent, a physiologically acceptable carrier, or a combination thereof.

In some embodiments, the treatment regimens described herein may be used before, after or in combination with other cancer therapies, including, but not limited to, surgery, cryosurgery, light therapy and hyperthermia therapy.

### Example 1: Metabolic Targeting Chemotherapy Has a Beneficial Clinical Effect (Comparative Data)

*Patients.* Six patients having various metastatic late stage cancers (i.e., stage IIIB/IV) that were previously unresponsive to standard treatment with one or more chemotherapeutic agents, radiation, surgery or other modalities of treatment were selected and underwent treatment as described below.

*Treatment Regimen.* Prior to treatment, patients were imaged by computed tomography (CT), magnetic resonance imaging (MRI), positron emission tomography (PET), or a combination thereof, and a baseline tumor mass was calculated. On day 0, patients were fasted overnight to reach baseline glucose levels (typically about 4.5-5.5 mmol/L). The following day (Day 1), after an initial intravenous flush with 500cc saline, all patients received insulin (Humalog) at a dose of 0.1 to 0.5 units/kg body weight intravenously (i.v.) to lower blood glucose levels to roughly half of the baseline level (about 2.2-2.8 mmol/L). Blood glucose levels were monitored for the duration of the subsequent treatment regimen.

After the desired blood glucose levels were reached, one or more chemotherapeutic agents were injected (i.v.) separately by a slow bolus or by dripping saline at an initial dosage that is 5-50% of the standard dosage for each particular agent. The dosage may be increased according to the patient's response to the initial dose or disease progression. After the chemotherapeutic treatment, patients were recovered from low blood glucose levels with an i.v. injection of a glucose solution (typically 50ml of 20% glucose solution) and were rehydrated with saline and consumption of liquids.

Patients were treated once or twice weekly with the treatment regimen described above for up to 18 total treatments. Table 2 (shown below) shows information and the specific chemotherapeutic agents used in the treatment regimen for each patient.

**Table 2. Treatment Regimens**

| **Patient No** | **Age/Sex/Wei ght** | **Type of Cancer** | **Chemotherapeutics used** (Dosage) | **# of Treatment s** |
|---|---|---|---|---|
| 1 | 56/M/62kg | Esophagea l | Gemcitabine (400mg) + THP-COHP* (30mg) + 5-fluorouracil (200mg) | 12 |
| | | | Gemcitabine (200mg) + THP-COHP* (25mg) + 5-fluorouracil (200mg) | 6 |
| | | | | **18 (TOTAL)** |
| 2 | 55/M/50kg | Colon | COHP* (50mg) + 5-fluorouracil (200mg) | 12 |
| | | | Mitomysin C (2mg) + vincristine (0.5mg) + 5-fluorouracil (200mg) | 6 |
| | | | | **18 (TOTAL)** |
| 3 | 51/F/60kg | Breast | Cyclophosphamide (200mg) + cisplatin (10mg) + doxorubicin (6mg) | 6 |
| | | | | **6 (TOTAL)** |
| 4 | 30/F/47kg | Lung | Cyclophosphamide (100mg) + vinorelbine (6mg) | 6 |
| | | | | **6 (TOTAL)** |
| 5 | 72/M/47kg | Esophagus | Etoposide (30mg) + oxaliplatin (30mg) | 12 |
| | | | Methotrexate (10mg) + oxaliplatin (30mg) + gemcitabine (200mg) | 6 |
| | | | | **18 (TOTAL)** |
| 6 | 47/F/50kg | Stomach | Etoposide (20mg) + pirarubicin (8mg) + 5-fluorouracil (200mg) | 18 |
| | | | | **18 (TOTAL)** |

*Evaluation of Tumor Response.* After treatment, patients were imaged by computed tomography (CT), magnetic resonance imaging (MRI), positron emission tomography (PET), or a combination thereof, to calculate a post-treatment tumor mass. A response category was assigned to each patient based on a comparison of the baseline tumor mass to the post-treatment tumor mass.

The Standard World Health Organization (WHO) criteria were used to determine the response to the treatment regimen described above. Briefly, a complete response (CR) category is assigned when no clinically detectable cancer is found after treatment. A partial response (PR) category is assigned when a decrease in measurable tumor mass is observed (≥50% decrease), no new areas of tumor develop, and no area of tumor shows progression. A minimal response (MR) category is assigned when a decrease in measurable tumor mass is observed (<50% decrease), no new areas of tumor develop, and no area of tumor shows progression. A progressive disease (PD) category is assigned when the mass of one or more tumor sites increases (>25% increase) or new lesions appear. A stable disease (SD) category is assigned when a measurable mass does not meet the criteria for CR, PR, MR or PD. A patient is considered to have received a clinical benefit as a result of a particular treatment regimen based an objective response of CR, PR, MR, or SD.

Table 3 shows tumor response to the treatment regimens described above. Briefly, patients 5 and 6 showed a partial response, patients 2 and 3 showed a minimal response, patient 1 showed stable disease and patient 4 showed progressive disease. Based on these results, 5/6 showed a clinical benefit of metabolic targeting therapy. This indicates that a treatment regimen that includes a decrease in blood glucose by administering a dose of insulin in combination with one or more chemotherapeutic agents results in an improvement over treatment with one or more chemotherapeutic agents alone in patients with late stage cancer.

**Table 3. Response to Treatment**

| **Patient No** | **Age/Sex/Wei ght** | **Type of Cancer** | **Assigned Response Category** | **Clinical Benefit?** |
|---|---|---|---|---|
| 1 | 56/M/62kg | Esophageal | SD | Yes |
| 2 | 55/M/50kg | Colon | MR | Yes |
| 3 | 51/F/60kg | Breast | MR | Yes |
| 4 | 30/F/47kg | Lung | PD | No |
| 5 | 72/M/47kg | Esophagus | PR | Yes |
| 6 | 47/F/50kg | Stomach | PR | Yes |

### Example 2: Metabolic Targeting Chemo-Immunotherapy for the Treatment of Cancer

*Patients.* Patients treated at Xi'an Xingcheng Borui Hospital and Yangling Demonstration Zone Hospital, having various metastatic late stage cancers (i.e., stage IIIB/IV; cancer type shown in Table 4 below) and were previously unresponsive to standard treatment with one or more chemotherapeutic agents, radiation, surgery or other modalities of treatment were selected to undergo treatment as described below.

*Treatment Regimen.* Prior to treatment, patients were imaged by computed tomography (CT), magnetic resonance imaging (MRI), positron emission tomography (PET), or a combination thereof, and a baseline tumor mass was calculated.

On day 0, patients were administered an immunologic agent such as a therapeutic antibody or functional fragment thereof. Therapeutic antibodies that will be used in this treatment regimen may include commercially available antibodies against EGFR or HER2 such as cetuximab (Erbitux®), panitumumab (Vectibix®) and trastuzumab (Herceptin®). The therapeutic antibody was administered to the patients at a dose that is lower than the standard dose typically given for cancer treatment. The dose is typically between about 1/2 to 1/1000^{th} of the standard dose. Although a single dose of the therapeutic antibody may be sufficient for the duration of the therapeutic regimen, optional additional booster doses may be given if needed. The booster dose may be repeated every two weeks, if needed.

Following treatment with the immunologic agent, the patients were fasted overnight to reach baseline glucose levels (typically about 4.5-5.5 mmol/L). The following day (Day 1), after an initial intravenous flush with 500cc saline, all patients received insulin (Humalog) at a dose of 0.1 to 0.5 units/kg body weight intravenously (i.v.) to lower blood glucose levels to roughly half of the baseline level (about 2.2-2.8 mmol/L). Blood glucose levels are monitored for the duration of the subsequent treatment regimen.

After the desired blood glucose levels are reached, one or more chemotherapeutic agents will be injected (i.v.) separately by a slow bolus or by dripping saline at a dosage that is 5-25% of the standard dosage for each particular agent. The one or more chemotherapeutic agents were selected based on the type of cancer and severity of the disease. For example, the agents may be any one or more chemotherapeutic agents in combination as described in Example 1 above (see patents 1-6 in Table 2), or may be one or more of the agents described in the disclosure above. After the chemotherapeutic treatment, patients will be recovered from low blood glucose levels with an injection (i.v.) of a glucose solution (typically 50ml of 20% glucose solution) and are rehydrated with saline and consumption of liquids.

Patients were treated once or twice weekly with the treatment regimen described above for up to 24 total treatments. The total number of treatments may vary due to clinical outcome or various other factors as determined by one skilled in the art. Table 4 below summarizes the treatment regimens used on each of the patients.

**Table 4. Treatment Regimens**

| **Patient No** | **Age/Sex/Weight** | **Type of Cancer** | **Chemotherapeutics used (Dosage)** | **# of Treatments** | **Erbitux (Dosage)** |
|---|---|---|---|---|---|
| 1 | 71/M/68kg | Lung | Vinorelbine (10mg)+ Cisplatin (10mg) | 12 | 1x20mg |
| 2 | 60/M/63kg | Stomach | Vinorelbine (10mg)+ Cisplatin (10mg)+ 5-fluorouracil (250mg) | 12 | 1x20mg |
| 3 | 52/F/49kg | Stomach | Vinorelbine (10mg) + Cisplatin (10mg) + 5-fluorouracil (250mg) | 12 | 1x20mg |
| 4 | 43/F/53kg | Breast | Vinorelbine (10mg) + Epirubicin (10mg) | 12 | 1x20mg |
| 5 | 47/M/70kg | Esophagus | Vinorelbine (10mg)+ Cisplatin (10mg)+ 5-fluorouracil (250mg) | 12 | 1x20mg |
| 6 | 62/M/65kg | Lung | Etoposide (100mg)+ Cisplatin (10mg) | 12 | 1x20mg |

*Evaluation of Tumor Response.* After treatment, patients were imaged by computed tomography (CT), magnetic resonance imaging (MRI), positron emission tomography (PET), or a combination thereof, to calculate a post-treatment tumor mass. A response category will be assigned to each patient based on a comparison of the baseline tumor mass to the post-treatment tumor mass.

The Standard World Health Organization (WHO) criteria will be used to determine the response to the treatment regimen described above. Briefly, a complete response (CR) category is assigned when no clinically detectable cancer is found after treatment. A partial response (PR) category is assigned when a decrease in measurable tumor mass is observed (≥50% decrease), no new areas of tumor develop, and no area of tumor shows progression. A minimal response (MR) category is assigned when a decrease in measurable tumor mass is observed (<50% decrease), no new areas of tumor develop, and no area of tumor shows progression. A progressive disease (PD) category is assigned when the mass of one or more tumor sites increases (>25% increase) or new lesions appear. A stable disease (SD) category is assigned when a measurable mass does not meet the criteria for CR, PR, MR or PD. A patient is considered to have received a clinical benefit as a result of a particular treatment regimen based an objective response of CR, PR, MR, or SD.

Table 5, below shows the response of each patient to the treatment regimen described above.

**Table 5. Response to Treatment**

| **Patient No** | **Age/Sex/Weight** | **Type of Cancer** | **Assigned Response Category** | **Clinical Benefit?** |
|---|---|---|---|---|
| 1 | 71/M/68kg | Lung | CR | Yes |
| 2 | 60/M/63kg | Stomach | CR | Yes |
| 3 | 52/F/49kg | Stomach | PR | Yes |
| 4 | 43/F/53kg | Breast | PR | Yes |
| 5 | 47/M/70kg | Esophagus | PR | Yes |
| 6 | 62/M/65kg | Lung | PR | Yes |

Results will be compared to the results in Example 1. Briefly, all patients showed a response and clinical benefit as a result of receiving the treatment regimen: two of which showed a complete response. Thus, patients treated with one or more immunologic agents (e.g., a therapeutic antibody) in addition to a metabolic targeting therapy show an increased clinical benefit as compared to the metabolic targeting therapy alone. This increased clinical benefit may be a result of a synergistic effect of the metabolic targeting of the cancer cells in combination with the low dose chemotherapeutic agents allowing a more efficient immune response to the Fc domain of the therapeutic antibodies.

Notably, Patient 4 showed a significant response to the treatment regimens described above. Patient 4 is a 43 year old female patient who presented with recurrent breast cancer 7 years post surgery and chemotherapy treatment. Wide spread metastasis was seen throughout the bones of the patient on a PET/CT scan (Figure 3). The Patient was administered 12 treatments of metabolic targeted chemo-immunotherapy over a period of 6 weeks. A PET/CT scan taken after treatment showed remarkable remission of bone metastasis (Figure 3, top panel).

The same patient presented with a large mass in the lower left lung measuring 1.3 X 1.5 X 0.8 cm in the pre-treatment PET/CT scan (Figure 4, bottom panel). SUV value was low, 1.5, indicating low glucose uptake in the lesion. A follow up PET/CT scan showed an increase of the left lung mass size, 1.3X1.5X1.8 cm, however a slight decrease in SUV, 1.3 (Figure 4, top panel), indicating that although the size increased, the metabolic state of the lesion was likely more stable, which may contribute to a reduction in the aggressiveness of the cancer. The results of these studies indicate that the metabolic targeting chemo-immunotherapy regimens described herein target metastatic cancer cells, in particular, the treatment methods described herein target cancer stem cells, which are likely the predominant cancer cell type responsible for metastasis and invasiveness of a cancer.

### REFERENCES

Abita, J. F., et al., 1984, Leukemia Res. 8:213.
Alabaster O., Vonderharr B.K., Shafie S.M. Metabolic modification by insulin enhances methotrexate cytoxicity in MCF-7 human breast cancer cells. Eur J Cancer Clin Oncol 1981; 17:1223-1228.
Al-Hajj M, Wicha MS, Benito-Hernandez A, Morrison SJ, Clarke MF. Prospective identification of tumorigenic breast cancer cells. Proceedings of the National Academy of Sciences USA 100:3983-3988, 2003.
Ayre, SG, Skaletski B., Mosnaim A.D. Blood-brain barrier passage of azidothymidine in rats: effect of insulin. Res Commun Chem Pathol Pharmacol 1989; 63:45-52.
Ayre, SG, Perez Garcia y Bellon D., Perez Garcia Jr., D. Insulin potentiation therapy: a new concept in the management of chronic degenerative disease. Med Hypotheses 1986; 20(2):199-210.
Ayre, SG, Perez Garcia y Bellon D., Perez Garcia Jr., D. Neoadjuvant low-dose chemotherapy with insulin in breast carcinomas. Eur J Cancer 1990; 26: 1262-1263.
Ayre, SG, et al., 2000, Medical Hypotheses 55:330.
Ayre, SG, New approaches to the delivery of drugs to the brain. Med Hypotheses 1989; 29:283-291.
Bonilla L, Ben-Aharon I, Vidal L, Gafter-Gvili A, Leibovici L, Stemmer SM., "Dose-dense chemotherapy in nonmetastatic breast cancer: a systematic review and meta-analysis of randomized controlled trials." J Natl Cancer Inst. 2010 Dec 15;102(24):1845-54.
Chan, Jennifer L., Katherine C. Tang, Anoop P. Patel, Larissa M. Bonilla, Nicola Pierobon, Nicholas M. Ponzio and Pranela Rameshwar, Antigen-presenting property of mesenchymal stem cells occurs during a narrow window at low levels of interferon-Gamma□□. Blood 107: 4817-4824, 2006.
Colman P.G., Harrison L.C. Structure of insulin/insulin-like growth factor-1 receptors on the insulinoma cell, RIM-m5F. Biochem Biophys Res Commun 1984;124:657-662.
Cullen J.K., Yee D., Sly W.S., et al. Insulin-like growth factor receptor expression and function in human breast cancer. Cancer Res 1990; 50:48-53.
Dean M, Fojo T, Bates S. Tumour stem cells and drug resistance. Nature Reviews Cancer 5:275-284, 2005.
Dean M. Cancer stem cells: Implications for Cancer Causation and Therapy Resistance, Discovery Medicine, 5(27):278-282, 2005
Dowling RJ, Goodwin PJ, Stambolic V., "Understanding the benefit of metformin use in cancer treatment." BMC Med. 2011 Apr 6;9:33.
Elstrom RL, Bauer DE, Buzzai M, Karnauskas R, Harris MH, Plas DR, Zhuang H, Cinalli RM, Alavi A, Rudin CM, Thompson CB., "Akt stimulates aerobic glycolysis in cancer cells." Cancer Res. 2004 Jun 1;64(11):3892-9.
Emerman J.T, Siemiatkowski J. Effects of endocrine regulation of growth of a mouse mammary tumor on its sensitivity to chemotherapy. Cancer Res 1984; 44: 1327-1332.
Eppenberger U. New aspects in the molecular growth regulation of mammary tumors. In: Eppenberger U., Goldhirsch A. eds. Recent Results in Cancer Research, Vol. 113: Endocrine Therapy and Growth Regulation of Breast Cancer. Berlin-Heidelberg, 1989, 1-3.
Gasparro FP, et al. Receptor-mediated photo-cytotoxicity: synthesis of a photoactivatable psoralen derivative conjugated to insulin. Biochem Biophys Res Comm 1986; 141:502-509.
Gottesman MM, Fojo T, Bates SE. Multi-drug resistance in cancer: role of ATP-dependent transporters. Nature Reviews Cancer 2:48-58, 2002.
Goustin A.S., Leof E.B., Shipley G.D., Moses H.L. Growth factors and cancer. Cancer Res 1986; 46:1015-1029.
Gross G.E., Boldt D.H., Osborne C.K. Perturbation by insulin of human breast cancer cell kinetics. Cancer Res 1984; 44:3570-3575.
Xianghui He, Tom C. Tsang, Brain L. Pipes, Richard J. Ablin and David T. Harris, "A Stem Cell Fusion Model of Carcinogenesis" Journal of Experimental Therapeutics and Oncology, Vol. 5, pp. 101-109, 2005.
Heddleston JM, Li Z, Lathia JD, Bao S, Hjelmeland AB, Rich JN., "Hypoxia inducible factors in cancer stem cells." Br J Cancer. 2010 Mar 2;102(5):789-95. Epub 2010 Jan 26
Hirsch HA, Iliopoulos D, Tsichlis PN, Struhl K., "Metformin selectively targets cancer stem cells, and acts together with chemotherapy to block tumor growth and prolong remission." Cancer Res. 2009 Oct 1;69(19):7507-11. Epub 2009 Sep 14. Erratum in: Cancer Res. 2009 Nov 15;69(22):8832.
Hilf R. The actions of insulin as a hormonal factor in breast cancer. In: Pike M.C, Siiteri P.K, Welsch C.W, eds. Hormones and Breast Cancer, Cold Spring Harbor Laboratory, 1981: 317-337.
Holdaway I.M., Freisen H.G. Hormone binding by human mammary carcinoma. Cancer Research 1977; 37:1946-1952.
Jacobs S, Cook S, Svoboda M, Van Wyk J.J. Interaction of the monoclonal antibodies alpha-IR-1 and alpha-IR3 with insulin and somatomedin-C receptors. Endocrinol 1986; 118:223-226.
Jaques G., Rotsch M., Wegmann C., et al. Production of immunoreactive insulin-like growth factor 1 and response to exogenous IGF-1 in small cell lung cancer cell lines. Exp Cell Res 1988;176: 336-343.
Jeffcoat R. and Jame A.T. The regulation of desaturation and elongation of fatty acids in mammals. Numa S. (Ed), Fatty Acid Metabolism and its Regulation. Elsevier Science Publishers BN. p.85-112, 1984
Karey K.P., Sirbasku D.A. Differential responsiveness of human breast cancer cell lines MCF-7 and T47D to growth factors and 17B-estradiol. Cancer Res 1988; 48:4083-4092.
Kiang D.T., Bauer G.E., Kennedy B.J. Immunoassayable insulin in carcinoma of the cervix associated with hypoglycemia. Cancer 1973; 31:801-804.
Koroljow, S. Two cases of malignant tumors with metastases apparently treated successfully with hypoglycemic coma. Psychiatric Quarterly 1962; 36(1):261-270.
Lee P.D.K., Rosenfeld R.G., Hintz R.L,. Smith S.D. Characterization of insulin, insulin-like growth factors I and II, and growth hormone receptors on human leukemic lymphoblasts. J Clin Endocr Metab 1986; 62: 28-35.
Leonard GD, Fojo T, Bates SE. The role of ABC trasporters in clinical practice. The Oncologist 2003; 8:411-424.
Lippman M.E., Dickson R.B., Kasid A., et al. Autocrine and paracrine growth regulation of human breast cancer. J Steroid Biochem 1986; 24:147-154.
Jeffcoat R. The biosynthesis of unsaturated fatty acids and its control in mammalian liver. Essays Biochem 1979;15:1-36.
Poznansky M.J., Singh R., Singh B., Fantus G. Insulin: Carrier potential for enzyme and drug therapy. Science 1984; 223:1304-1306.
Mountjoy K.G., Holdaway I.M., Finlay G.J. Insulin receptor regulation in cultured human tumor cells. Cancer Research 1983; 43:4537-4542.
Myal Y., Shiu R.P.C., Bhomick B., Bala B. Receptor binding and growth promoting activity of insulin-like growth factors in human breast cancer cells [T-47D] in culture. Cancer Res 1984; 44:5486-5490.
Nakanishi Y., Mulshine J.L., Kasprzyk P.G., et al. Insulin-like growth factor-1 can mediate autocrine proliferation of human small cell lung cancer cell lines in vitro. J Clin Invest 1988; 82: 354-359.
Neufeld, O. Insulin therapy in terminal cancer: a preliminary report. J Amer Geriatric Soc 1962; 10(3):274-6.
Ohgimoto K, Ohgimoto S, Ihara T, Mizuta H, Ishido S, Ayata M, Ogura H, Hotta H. "Difference in production of infectious wild-type measles and vaccine viruses in monocyte-derived dendritic cells". Virus Res 123 (1): 1-8, 2007.
Oleesky S., Bailey I., Samos S., Bilkus D. A fibrosarcoma with hypoglycemia and a high serum insulin level. Lancet 1962; 2:378-380.
Oster J.B., Creasey W.A. Enhancement of cellular uptake of ellipticine by insulin preincubation. Eur J Cancer Clin Oncol 1981; 17:1097-1103.
Papa V., Milazzo G., Goldfine I.D., Waldman F.M., Vigneri R. Sporadic amplification of the insulin receptor gene in human breast cancer. J Endocrinol Invest 1997; 20(9):531-6.
Papa V., Pezzino V., Costantino A., et al. Elevated insulin receptor content in human breast cancer. J Clin Invest 1990; 86:1503-1510.
Pardridge W.M., Eisenberg J., Yang J. Human blood-brain barrier insulin receptor. J Neurochem 1985; 44:1771-1778.
Pavelik L., Pavelik K., Vuk-Pavlovic S. Human mammary and bronchial carcinomas: in vivo and in vitro secretion of substances immunologically cross-reactive with insulin. Cancer 1984; 53(11):2467-2471.
Pavelik K., Bolanca M., Vecek N., et al. Carcinomas of the cervix and corpus uteri in humans: stage-dependent blood levels of substance(s) immunologically cross-reactive with insulin. J Natl Cancer Inst 1992; 68:891-894.
Pavelic K., Popovic M. Insulin and glucagon secretion by renal adenocarcinoma. Cancer 1981; 48:98-100.
Pavelic K., Odavic M., Pekic B., et al. Correlation of substance(s) immunologically cross-reactive with insulin, glucose and growth hormone in Hodgkin's lymphoma patients. Cancer Lett 1982; 17:81-86.
Pelicano H., Martin DS, Xu R-H, Huang P. Glycolysis inhibition for anticancer treatment. Oncogene 2006; 25:4633-4646.
Quinn K.A., Treston A.M., Unsworth E.J. et al. Insulin-like growth factor expression in human cancer cell lines. J Biol Chem 1996; 271:11477-83.
Raffaghello L, Lee C, Safdie FM, Wei M, Madia F, Bianchi G, Longo VD., "Starvation-dependent differential stress resistance protects normal but not cancer cells against high-dose chemotherapy." Proc Natl Acad Sci USA. 2008 Jun 17;105(24):8215-20. Breast Cancer Res Treat 1998; 47(3):219-33.
Schilsky R.L., Bailey B.D., Chabner B.A. Characteristics of membrane transport of methotrexate by cultured human breast cancer cells. Biochem Pharmacol 1981; 30:1537-1542.
Shames J.M., Dhurandhar N.R., Blackard W.G. Insulin-secreting bronchial carcinoid tumor with widespread metastases. Am J Med 1968; 44:632-637.
Shinitzky M., Henkart P. Fluidity of cell membranes - current concepts and trends. Int Rev Cytol 1971: 60:121-147.
Simsek T, Kocabas F, Zheng J, Deberardinis RJ, Mahmoud Al, Olson EN, Schneider JW, Zhang CC, Sadek HA, "The distinct metabolic profile of hematopoietic stem cells reflects their location in a hypoxic niche." Cell Stem Cell. 2010 Sep 3;7(3):380-90.
Spring-Mills E., Stearns S.B., Smith T.H., et al. Immunoreactive hormones in human breast tissues. Surgery 1983; 94(6):946-950.
Sporn M.B., Todaro G.J. Autocrine secretion and malignant transformation of cells. N Engl J Med 1980; 308:487-490.
Surmacz E., Guvakova M.A., Nolan M.K., Nicosia R.F., Sciacca L. Type I insulin-like growth factor receptor function in breast cancer. Breast Cancer Res Treat 1998; 47(3):255-67.
Van der Burg B., de Laat S.W., van Zoelen E.J.J. Mitogenic stimulation of human breast cancer cells in a growth-factor defined medium: synergistic action of insulin and estrogens. In: Brescani F, King R.J.B, Lippman, M.E, Raynaud J.P, eds. Progress in Cancer Research and Therapy, vol. 35: Hormones and Cancer 3. New York, Raven Press, Ltd., 1988: 231-233.
Van Wyk J.J., Graves C.D., Casella S.J., Jacobs, S. Evidence from monoclonal antibody studies that insulin stimulates deoxyribonucleic acid synthesis through the type I somatomedin receptor. J Clin Endocrinol Metab 1985; 61:639-643.
Warburg O. The metabolism of carcinoma cells. J Cancer Res 1925; 9:148-163.
Weber, Wolfgang A., "Use of PET for Monitoring Cancer Therapy and for Predicting Outcome" J Nucl Med. 2005 Jun;46(6):983-95.
Winquist RJ, Boucher DM, Wood M, Furey BF., "Targeting cancer stem cells for more effective therapies: Taking out cancer's locomotive engine." Biochem Pharmacol. 2009 Aug 15;78(4):326-34.
Wong M., Holdaway I.M. Insulin binding by normal and neoplastic colon tissue. Int J Cancer 1985; 35:335-341.
Workman, Ronald B Jr. and Coleman, R. Edward, "PET/CT Essentials for Clinical Practice" Springer Science+Business Media, LLC, 2006.
Xu RH, Pelicano H, Zhou Y, Carew JS, Feng L, Bhalla KN, Keating MJ, Huang P., "Inhibition of glycolysis in cancer cells: a novel strategy to overcome drug resistance associated with mitochondrial respiratory defect and hypoxia." Cancer Res. 2005 Jan 15;65(2):613-21.
Yee D. The insulin-like growth factors and breast cancer- revisited. Breast Cancer Res Treat 1998; 47(3):197-9.
Yoshimasa S., et al. A new approach to the detection of autoantibodies against insulin receptors that inhibit the internalization of insulin into human cells. Diabetes 1984; 33:1051-1054.
Zapf J., Froesch E.R. Insulin-like growth factors/somatomedins: structure, secretion, biological actions and physiological role. Hormone Res 1986; 24:121-130.

## Claims

1. A metabolic targeting chemo-immunotherapy regimen for use in the treatment of cancer, the metabolic targeting chemo-immunotherapy regimen comprising:
cetuximab at a dose of 20 mg;
insulin at a dose of 0.1 to 0.5 units/kg to reduce blood glucose level to 2.2 to 2.8 mmol/L; and
a therapeutically effective dose of one or more chemotherapeutic agents, to be administered at a blood glucose level of 2.2 to 2.8 mmol/L.

2. The metabolic targeting chemo-immunotherapy regimen for use according to claim 1, further comprising administering one or more booster doses of cetuximab.

3. The metabolic targeting chemo-immunotherapy regimen for use according to claim 1 or 2, wherein the one or more chemotherapeutic agents are administered daily, every other day, 3 times weekly, or biweekly.

4. The metabolic targeting chemo-immunotherapy regimen for use according to any one of claims 1 to 3, wherein insulin is used in combination with fasting.

5. The metabolic targeting chemo-immunotherapy regimen for use according to any one of claims 1 to 4, wherein the one or more chemotherapeutic agents are selected from the group consisting of alkylating agents, antimetabolites, anti-tumor antibiotics, topoisomerase inhibitors, mitotic inhibitors, hormone therapy, glycolysis inhibitors, targeted therapeutics and immunotherapeutics.

6. The metabolic targeting chemo-immunotherapy regimen for use according to any one of claims 1 to 5, wherein the metabolic targeting chemo-immunotherapy is used for treating a cancer selected from the group consisting of, esophageal cancer, breast cancer, lung cancer, bone cancer, bladder cancer, brain cancer, cancer of the urinary tract, carcinoma, cervical cancer, colon cancer, gastric cancer, head and neck cancer, hepatocellular cancer, liver cancer, lymphoma and leukemia, melanoma, ovarian cancer, pancreatic cancer, pituitary cancer, prostate cancer, rectal cancer, renal cancer, sarcoma, testicular cancer, thyroid cancer, and uterine cancer.

## Patentansprüche

1. Ein metabolisch abzielendes Chemo-Immuntherapie-Regimen zur Verwendung in der Behandlung von Krebs, wobei das metabolisch abzielende Chemo-Immuntherapie-Regimen umfasst:
Cetuximab in einer Dosis von 20 mg;
Insulin in einer Dosis von 0,1 bis 0,5 Einheiten/kg, um den Blutglukosespiegel auf 2,2 bis 2,8 mmol/L zu senken; und
eine therapeutisch wirksame Dosis von einem oder mehreren chemotherapeutischen Mitteln, zu verabreichen bei einem Blutglukosespiegel von 2,2 bis 2,8 mmol/L.

2. Das metabolisch abzielende Chemo-Immuntherapie-Regimen zur Verwendung gemäß Anspruch 1, ferner umfassend das Verabreichen einer oder mehrerer Verstärkerdosen an Cetuximab.

3. Das metabolisch abzielende Chemo-Immuntherapie-Regimen zur Verwendung gemäß Anspruch 1 oder 2, wobei das eine oder mehrere chemotherapeutische Mittel täglich, jeden zweiten Tag, 3 Mal pro Woche oder zweiwöchentlich verabreicht wird.

4. Das metabolisch abzielende Chemo-Immuntherapie-Regimen zur Verwendung gemäß einem der Ansprüche 1 bis 3, wobei Insulin in Kombination mit Fasten verwendet wird.

5. Das metabolisch abzielende Chemo-Immuntherapie-Regimen zur Verwendung gemäß einem der Ansprüche 1 bis 4, wobei das eine oder mehrere chemotherapeutische Mittel ausgewählt sind aus der Gruppe bestehend aus Alkylierungsmitteln, Antimetaboliten, anti-Tumor-Antibiotika, Topoisomeraseinhibitoren, mitotischen Inhibitoren, Hormontherapie, Glykolyseinhibitoren, zielgerichtete Therapeutika und Immunotherapeutika.

6. Das metabolisch abzielende Chemo-Immuntherapie-Regimen zur Verwendung gemäß einem der Ansprüche 1 bis 5, wobei die metabolisch abzielende Chemo-Immuntherapie verwendet wird, um Krebs, ausgewählt aus der Gruppe bestehend aus Speiseröhrenkrebs, Brustkrebs, Lungenkrebs, Knochenkrebs, Blasenkrebs, Gehirnkrebs, Krebs des Harntraktes, Karzinom, Gebärmutterhalskrebs, Darmkrebs, Magenkrebs, Kopf- und Nackenkrebs, Leberzellkrebs, Leberkrebs, Lymphom und Leukämie, Melanom, Eierstockkrebs, Bauchspeicheldrüsenkrebs, Hypophysenkrebs, Prostatakrebs, Enddarmkrebs, Nierenkrebs, Sarkomen, Hodenkrebs, Schilddrüsenkrebs und Uteruskrebs, zu behandeln.

## Revendications

1. Schéma chimio-immunothérapeutique de ciblage métabolique destiné à une utilisation dans le traitement d'un cancer, le schéma chimio-immunothérapeutique de ciblage métabolique comprenant :
du cétuximab à une dose de 20 mg ;
de l'insuline à une dose de 0,1 à 0,5 unité/kg pour réduire le taux de glycémie à 2,2 à 2,8 mmol/l ; et
une dose thérapeutiquement efficace d'un ou plusieurs agents chimiothérapeutiques à administrer à un taux de glycémie de 2,2 à 2,8 mmol/l.

2. Schéma chimio-immunothérapeutique de ciblage métabolique destiné à une utilisation selon la revendication 1, comprenant en outre l'administration d'une ou de plusieurs doses de rappel de cétuximab.

3. Schéma chimio-immunothérapeutique de ciblage métabolique destiné à une utilisation selon la revendication 1 ou 2, dans lequel le ou les agents chimiothérapeutiques sont administrés quotidiennement, tous les deux jours, 3 fois par semaine ou deux fois par semaine.

4. Schéma chimio-immunothérapeutique de ciblage métabolique destiné à une utilisation selon l'une quelconque des revendications 1 à 3, dans lequel l'insuline est utilisée en combinaison avec le jeûne.

5. Schéma chimio-immunothérapeutique de ciblage métabolique destiné à une utilisation selon l'une quelconque des revendications 1 à 4, dans lequel le ou les agents chimiothérapeutiques sont choisis dans le groupe constitué par les agents alkylants, les antimétabolites, les antibiotiques antitumoraux, les inhibiteurs de la topoisomérase, les inhibiteurs mitotiques, l'hormonothérapie, les inhibiteurs de la glycolyse, les agents thérapeutiques et immunothérapeutiques ciblés.

6. Schéma chimio-immunothérapeutique de ciblage métabolique destiné à une utilisation selon l'une quelconque des revendications 1 à 5, dans lequel la chimio-immunothérapeutie de ciblage métabolique est utilisée pour le traitement d'un cancer choisi dans le groupe constitué par un cancer de l'oesophage, un cancer du sein, un cancer du poumon, un cancer des os, un cancer de la vessie, un cancer du cerveau, un cancer du tractus urinaire, un carcinome, un cancer du col de l'utérus, un cancer du côlon, un cancer gastrique, un cancer de la tête et du cou, un cancer hépatocellulaire, un cancer du foie, un lymphome et une leucémie, un mélanome, un cancer de l'ovaire, un cancer du pancréas, un cancer de l'hypophyse, un cancer de la prostate, un cancer rectal, un cancer du rein, un sarcome, un cancer des testicules, un cancer de la thyroïde et un cancer de l'utérus.
